# EUROPÄISCHE PATENTSCHRIFT

(11) **EP 4 023 198 B1**
(45) Veröffentlichungstag und Bekanntmachung des Hinweises auf die Patenterteilung: **28.06.2023**
(21) Anmeldenummer: 21213440.7
(22) Anmeldetag: 09.12.2021
(51) Int. Cl.: A61F 13/496, A61F 13/49

(54) **INKONTINENZARTIKEL IN HÖSCHENFORM FÜR DIE AUFNAHME VON KÖRPERAUSSCHEIDUNGEN**
INCONTINENCE ARTICLES IN THE SHAPE OF PANTIES FOR THE COLLECTION OF BODILY FLUIDS
ARTICLE POUR L'INCONTINENCE EN FORME DE CULOTTE POUR LA COLLECTE DES EXCRÉTIONS CORPORELLES

(30) Priorität: 30.12.2020 DE 102020135136
(43) Veröffentlichungstag der Anmeldung: 06.07.2022
(73) Patentinhaber: Paul Hartmann AG, 89522 Heidenheim (DE)
(72) Erfinder: Bährle, Christian, 89542 Herbrechtingen (DE); Beyrle, Andreas, 89564 Nattheim (DE); Buch, Tamara, 89077 Ulm (DE); Eilers, Jörg, 89179 Beimerstetten (DE)
(74) Vertreter: DREISS Patentanwälte PartG mbB

(56) Entgegenhaltungen:
- EP-B1- 2 849 694
- EP-B1- 2 849 696
- WO-A1-2018/106160
- WO-A1-2018/123237

## Beschreibung

Die vorliegende Erfindung betrifft einen Inkontinenzartikel in Höschenform mit den Merkmalen des Oberbegriffs des Patentanspruchs 1.

Ein derartiger Artikel ist bekannt aus EP 2 849 696 B1.

Inkontinenzartikel in Höschenform unterscheiden sich prinzipiell von öffenbaren und schließbaren Inkontinenzartikeln in üblicher Windelform dadurch, dass durch die Höschenform der Hüftumfang schon vorgegeben ist und die Anpassung an unterschiedliche Körpergrößen ausgehend von einer Anzahl von Grundgrößen durch eine elastische Dehnbarkeit des Artikels erreicht wird. Hierfür werden in der Regel Elastifizierungsmittel, insbesondere in Form von Bändern oder Fäden, häufig als Lycra-Fäden bezeichnet, in vorgedehntem Zustand (Stretch-Bond-Verfahren) mit Chassismaterialien des Inkontinenzartikels, welche den Bauchabschnitt und den Rückenabschnitt bilden, verbunden, das heißt, sie werden in vorgedehntem Zustand an den Chassismaterialien z.B. mittels Kleber fixiert. Infolge ihrer Vorspannung raffen diese Elastifizierungsmittel die Chassismaterialien zusammen und bilden dabei Fältelungen, die typischerweise quer zur Vorspannungsrichtung der Elastifizierungsmittel, hier also in Längsrichtung des Artikels, verlaufen. Der Inkontinenzartikel bzw. die elastifizierten Chassismaterialien des Inkontinenzartikels lassen sich dann wieder elastisch dehnen, wenn der Inkontinenzartikel wie ein Höschen an den Benutzer angelegt wird, so dass die elastifizierten Chassismaterialien die Körperform adaptieren können. Anstelle von faden- oder bandförmigen Elastifizierungsmitteln können aber auch elastische Flächenmaterialien, wie insbesondere elastisch dehnbare Folienwerkstoffe, als Chassissmaterialien eingesetzt werden.

Zur unlösbaren Verbindung des Bauchabschnitts bzw. des Rückenabschnitts mit dem Schrittabschnitt im jeweiligen Überlappungsbereich könnte an sich grundsätzlich eine vollflächige oder eine nicht vollflächige Klebeverbindung gewählt werden. Häufig wird zum Fügen großflächiger Bereiche bei vorbekannten Hygieneartikeln ein vollflächiger Kleberauftrag oder ein im Wesentlichen die gesamte Fläche erfassender spiralförmiger, durch Düsen aufgebrachter Kleberauftrag verwendet. Es gibt aber auch Hygieneartikel, bei denen ein anderer Weg beschritten wird; so lehrt beispielsweise US 2004/0116886 A1, keine großflächigen Gebiete des jeweiligen Überlappungsbereichs zu fügen; auf diese Weise soll die Gestalt und Flexibilität der Komponenten im Überlappungsbereich frei bleiben und jedenfalls nicht durch eine Fügeverbindung nachteilig beeinflusst werden. Mit der vorgenannten EP 2 849 696 B1 wurde bereits der Vorschlag unterbreitet, im Wesentlichen den gesamten jeweiligen Überlappungsbereich mit der Kleberverbindung zu erfassen, jedoch zumindest bereichsweise eine streifenförmige Kleberverbindung mit Kleberstreifen und kleberfreien Streifen vorzusehen.

Bei typischen Inkontinenzartikeln in Höschenform der hier in Rede stehenden Art erstreckt sich der Schrittabschnitt sowohl in der Längsrichtung als auch in der Querrichtung deutlich über seitliche Längsränder und ein jeweiliges Längsende des Absorptionskörpers hinaus. In orthogonaler Projektion des Absorptionskörpers auf den Überlappungsbereich bzw. den Bauchabschnitt im eben ausgebreiteten Zustand des Bauchabschnitts befindet sich daher die Kleberverbindung und damit ein Kleberauftrag zwischen Schrittabschnitt und Bauchabschnitt typischerweise im gesamten Bereich der flächenhaften Erstreckung des Absorptionskörpers. Mit der vorliegenden Erfindung wurde festgestellt, dass es bei seitlichen Randbereichen von Absorptionskörpern, wo in der Regel ein sehr viel geringeres Flächengewicht an absorbierenden Materialien vorhanden ist als weiter mittig, gerade im Bereich des Bauchabschnitts, wo sich Bewegungen der Leistenbeuge bemerkbar machen, zu einem Verlust an Tragekomfort kommt, weil sie zu Verhärtungen oder zur Bildung von Hartstellen neigen, was bei mobilen Bewegungen des Benutzers in noch stärkerem Maße auftritt und verstärkt wahrgenommen wird.

Der vorliegenden Erfindung liegt die Aufgabe zugrunde, einen Inkontinenzartikel in Höschenform der eingangs genannten Art im Hinblick auf den Tragekomfort im Bereich des Bauchabschnitts noch weitergehend zu verbessern.

Diese Aufgabe wird bei einem Inkontinenzartikel der eingangs genannten Art erfindungsgemäß dadurch gelöst, dass die erste Quererstreckung (Q1) des hüftseitigen ersten Bereichs größer ist als die zweite Quererstreckung (Q2) des zweiten Bereichs und größer ist als die dritte Quererstreckung (Q3) des dritten Bereichs,
dass die dritte Quererstreckung (Q3) des dritten Bereichs größer ist als die zweite Quererstreckung (Q2) des zweiten Bereichs,
dass die zweite Quererstreckung (Q2) des zweiten Bereichs geringer ist als eine maximale Quererstreckung (Qsk) des Absorptionskörpers bei dem zweiten Bereich, und
dass der dritte Bereich in orthogonaler Projektion auf den Überlappungsbereich betrachtet in der Längsrichtung und in der Querrichtung vollständig innerhalb einer Erstreckung des Absorptionskörpers angeordnet ist und
dass der zweite Bereich in orthogonaler Projektion auf den Überlappungsbereich betrachtet zumindest in der Querrichtung vollständig innerhalb der Erstreckung des Absorptionskörpers angeordnet ist, so dass bauchseitige Eckbereiche des Absorptionskörpers in orthogonaler Projektion auf den Überlappungsbereich außerhalb des zweiten Bereichs liegen.

Mit der vorliegenden Erfindung wurde nicht nur das der Erfindung zugrunde liegende Problem erkannt, sondern es wurde zudem erkannt, dass der Verlust an Tragkomfort nicht wie zumeist angenommen lediglich eine Folge einer als unangenehm empfundenen Raffung von Chassismaterialien ist, sondern dass dies auf das Vorhandensein von Klebermaterialien zwischen Schrittabschnitt und Bauchabschnitt im Bereich von seitlichen Randbereichen und besonders Eckbereichen des Absorptionskörpers im Bauchabschnitt zurückzuführen ist oder zumindest hierdurch verstärkt wird. Ausgehärtete Klebermaterialien stellen per se Hartstellen dar, die sich, wenn sie infolge von Elastifizierungsmitteln zusammengezogen werden oder infolge von Bewegungen verstärkt lokalisiert werden, als Reibstellen mit der Körperoberfläche ausbilden können, was sehr rasch zu unangenehmen Begleiterscheinungen und insbesondere zu Rötungen, zum Einschneiden bis hin zu einer Schmerzempfindung führen kann.

Mit der vorliegenden Erfindung wird vorgeschlagen, dass sich die Kleberverbindung im zweiten Bereich und im dritten Bereich in der Querrichtung nicht bis zu seitlichen Längsrändern des Absorptionskörpers erstreckt und dass im zweiten Bereich ein Querabstand zu seitlichen Längsrändern des Absorptionskörpers vorhanden ist. Auf diese Weise ist in seitlichen Längsrandbereichen und insbesondere in bauchseitigen Eckbereichen des Absorptionskörpers in der Projektion auf den Bauchabschnitt kein Klebermaterial zwischen Schrittabschnitt und Bauchabschnitt vorhanden, welches zu Verhärtungen im Bereich des dort typischerweise dünnen Absorptionskörpers führen könnte. Es wird im Patentanspruch Bezug genommen auf eine maximale Quererstreckung (Qsk) des Absorptionskörpers bei diesem zweiten Bereich, weil Absorptionskörper typischerweise mit abgerundeten Ecken nach innen verlaufenden Rand aufweisen, was nicht ausgeschlossen werden sollte. Jedenfalls sollen bauchseitige Eckbereiche des Absorptionskörpers in orthogonaler Projektion auf den Überlappungsbereich von Schrittabschnitt und Bauchabschnitt außerhalb des zweiten Bereichs der Kleberverbindung liegen.

Es erweist sich als vorteilhaft, wenn in der Querrichtung zwischen dem zweiten Bereich und jeweiligen Längsrändern des Absorptionskörpers beidseits in der Querrichtung ein über die zweite Längserstreckung (L2) des zweiten Bereichs gleichbleibender oder variierender Querabstand (R2) vorgesehen ist und wenn wenigstens abschnittsweise über die zweite Längserstreckung (L2) des zweiten Bereichs dieser Querabstand (R2) in der Querrichtung wenigstens 15 mm, insbesondere wenigstens 20 mm, insbesondere wenigstens 25 mm, insbesondere wenigstens 30 mm, und insbesondere höchstens 80 mm, insbesondere höchstens 70 mm, insbesondere höchstens 60 mm, insbesondere höchstens 50 mm beträgt. Auch bei dieser Formulierung wird ein nach-innen-Laufen des Rands der bauchseitigen Eckbereiche des Absorptionskörpers bis zu dem zweiten Bereich der Kleberverbindung nicht ausgeschlossen, aber dennoch sichergestellt, dass hinreichend große Eckbereiche nicht in der Projektion oberhalb von Kleberhartstellen zu liegen kommen.

In weiterer Ausbildung der Erfindung erweist sich als vorteilhaft, wenn in der Querrichtung zwischen dem zweiten Bereich und jeweiligen Längsrändern des Absorptionskörpers beidseits in der Querrichtung ein über die zweite Längserstreckung (L2) des zweiten Bereichs gleichbleibender oder variierender Querabstand (R2) vorgesehen ist und wenn wenigstens abschnittsweise über die zweite Längserstreckung (L3) des zweiten Bereichs ein Verhältnis dieses Querabstands (R2) zu einer maximalen Quererstreckung Qsk des Absorptionskörpers bei dem zweiten Bereich jeweils wenigstens 0,14, insbesondere wenigstens 0,15, insbesondere wenigstens 0,16, und insbesondere höchstens 0,40, insbesondere höchstens 0,30 insbesondere höchstens 0,20 beträgt.

Weiter erweist es sich als besonders vorteilhaft, wenn in der Querrichtung zwischen dem dritten Bereich und jeweiligen Längsrändern des Absorptionskörpers beidseits in der Querrichtung ein über die dritte Längserstreckung (L3) des dritten Bereichs gleichbleibender oder variierender Querabstand (R3) vorgesehen ist und wenn wenigstens abschnittsweise über die dritte Längserstreckung (L3) des dritten Bereichs dieser Querabstand (R3) wenigstens 5 mm, insbesondere wenigstens 8 mm, insbesondere wenigstens 10 mm, insbesondere höchstens 30 mm, insbesondere höchstens 25 mm, insbesondere höchstens 20 mm beträgt. Somit wird vorgeschlagen, dass auch der dritte Bereich der Klebeverbindung in Querrichtung beabstandet ist zu jeweiligen Längsrändern des Absorptionskörpers, so dass ein Längsrandbereich des Absorptionskörpers in der Querrichtung außerhalb des Kleberauftrags angeordnet ist oder zu liegen kommt.

In Weiterbildung dieses Erfindungsgedankens erweist sich als vorteilhaft, wenn in der Querrichtung zwischen dem dritten Bereich und jeweiligen Längsrändern des Absorptionskörpers beidseits in der Querrichtung ein über die dritte Längserstreckung (L3) des dritten Bereichs gleichbleibender oder variierender Querabstand (R3) vorgesehen ist und wenn wenigstens abschnittsweise über die dritte Längserstreckung (L3) des dritten Bereichs ein Verhältnis dieses Querabstands (R3) zu einer maximalen Quererstreckung (Qsk) des Absorptionskörpers bei dem dritten Bereich jeweils wenigstens 0,04, insbesondere wenigstens 0,05, insbesondere wenigstens 0,06, und insbesondere höchstens 0,20, insbesondere höchstens 0,15, insbesondere höchstens 0,10 beträgt.

Wie schon ausgeführt ist der erste Bereich der Klebeverbindung vollständig außerhalb des Absorptionskörpers angeordnet. Um dort eine hinreichende flächenhaft erstreckte Verbindung zwischen dem Schrittabschnitt und dem Bauchabschnitt zu erzielen erweist es sich als vorteilhaft, wenn ein Verhältnis der ersten Quererstreckung (Q1) des ersten Bereichs zur zweiten Quererstreckung (Q2) des zweiten Bereichs wenigstens 1,6, insbesondere wenigstens 1,7, insbesondere wenigstens 1,8, insbesondere höchstens 2,5, insbesondere höchstens 2,4, insbesondere höchstens 2,3 beträgt.

Weiter erweist es sich als vorteilhaft, wenn ein Verhältnis der ersten Quererstreckung (Q1) des ersten Bereichs zur dritten Quererstreckung (Q3) des dritten Bereichs wenigstens 1,2, insbesondere wenigstens 1,3, insbesondere wenigstens 1,4, insbesondere höchstens 2,0, insbesondere höchstens 1,9, insbesondere höchstens 1,8, insbesondere höchstens 1,7 beträgt.

Weiter erweist es sich als vorteilhaft, wenn ein Verhältnis der zweiten Quererstreckung (Q2) des zweiten Bereichs zur dritten Quererstreckung (Q3) des dritten Bereichs wenigstens 0,6, insbesondere wenigstens 0,7, insbesondere höchstens 0,9, insbesondere höchstens 0,8 beträgt.

Wiederum im Hinblick auf die Erzielung einer hinreichenden flächenhaft erstreckten Verbindung zwischen dem Schrittabschnitt und dem Bauchabschnitt, und zwar entlang der Längsrichtung, erweist sich als vorteilhaft, wenn ein Verhältnis der ersten Längserstreckung (L1) des ersten Bereichs, die entlang der Längsmittelachse bestimmt ist, zur Längserstreckung (Lü) des Überlappungsbereichs von Schrittabschnitt und Bauchabschnitt, die entlang der Längsmittelachse (44) bestimmt ist, wenigstens 0,10, insbesondere wenigstens 0,12, insbesondere wenigstens 0,15 und insbesondere höchstens 0,50, insbesondere höchstens 0,45, insbesondere höchstens 0,40 beträgt. Sofern der erste Bereich mit über seine Quererstreckung konstanter erster Längserstreckung ausgebildet ist, also insbesondere rechteckförmig ausgebildet ist, kann die Längserstreckung natürlich an jeder Stelle ermittelt werden.

Es erweist sich weiter als vorteilhaft, wenn ein Verhältnis der zweiten Längserstreckung (L2) des zweiten Bereichs, die entlang der Längsmittelachse bestimmt ist, zur Längserstreckung (Lü) des Überlappungsbereichs von Schrittabschnitt und Bauchabschnitt, die entlang der Längsmittelachse bestimmt ist, wenigstens 0,20, insbesondere wenigstens 0,25, insbesondere wenigstens 0,30 und insbesondere höchstens 0,60, insbesondere höchstens 0,55, insbesondere höchstens 0,50 beträgt.

Und schließlich erweist sich als vorteilhaft, wenn ein Verhältnis der dritten Längserstreckung (L3) des dritten Bereichs, die entlang der Längsmittelachse bestimmt ist, zur Längserstreckung (Lü) des Überlappungsbereichs von Schrittabschnitt und Bauchabschnitt, die entlang der Längsmittelachse bestimmt ist, wenigstens 0,15, insbesondere wenigstens 0,18, insbesondere wenigstens 0,20 und insbesondere höchstens 0,50, insbesondere höchstens 0,45, insbesondere höchstens 0,40 beträgt.

Bei einer bevorzugten Ausführungsform sind die Bereiche der Kleberverbindung rechteckförmig ausgebildet und mit ihrer längeren Seite in der Querrichtung orientiert angeordnet. Wenn hingegen die Längserstreckung eines oder mehrerer der Bereiche über deren Quererstreckung variiert, dann beziehen sich die vorausgehend beanspruchten Werte und Verhältnisse auf die Längserstreckung entlang der Längsmittelachse, also im Symmetriezentrum des Inkontinenzartikels. An dieser Stelle bzw. in einem die Längsmittelachse einschließenden in der Längsrichtung erstreckten gedachten bandförmigen Abschnitt im Überlappungsbereich zwischen Schrittabschnitt und Bauchabschnitt aber auch zwischen Schrittabschnitt und Rückenabschnitt ist die Krafteinleitung auf die Fügeverbindung in leicht nachvollziehbarer Weise am größten, insbesondere schwerkraftbedingt im flüssigkeitsbeladenen Zustand des Absorptionskörpers.

Im Hinblick auf die Verteilung der jeweiligen Längserstreckung des zweiten und dritten Bereichs auf die Längserstreckung des Absorptionskörpers erweist sich als vorteilhaft, wenn ein Verhältnis der zweiten Längserstreckung (L2) des zweiten Bereichs, die entlang der Längsmittelachse bestimmt ist, zur Längserstreckung (Lsk) ein des Absorptionskörpers im Überlappungsbereich des Schrittabschnitts mit dem Bauchabschnitt, die entlang der Längsmittelachse bestimmt ist, wenigstens 0,35, insbesondere wenigstens 0,40, insbesondere wenigstens 0,45 und insbesondere höchstens 0,80, insbesondere höchstens 0,75, insbesondere höchstens 0,70 beträgt. Je größer dieses Verhältnis, desto größer gestalten sich die kleberfreien Eckbereiche des Absorptionskörpers.

Und es erweist sich als vorteilhaft, wenn ein Verhältnis der dritten Längserstreckung (L3) des dritten Bereichs, die entlang der Längsmittelachse bestimmt ist, zur Längserstreckung (Lsk) des Absorptionskörpers im Überlappungsbereich des Schrittabschnitts mit dem Bauchabschnitt, die entlang der Längsmittelachse bestimmt ist, wenigstens 0,25, insbesondere wenigstens 0,30, insbesondere wenigstens 0,35 und insbesondere höchstens 0,65, insbesondere höchstens 0,60, insbesondere höchstens 0,55 beträgt. Je kleiner dieses Verhältnis, desto größer gestalten sich die kleberfreien Eckbereiche des Absorptionskörpers.

Da der erste Bereich der Kleberbeschichtung außerhalb der flächenhaften Erstreckung des Absorptionskörpers angeordnet ist, erweist es sich als vorteilhaft, wenn der erste Bereich der Kleberbeschichtung einen in der Längsrichtung und in der Querrichtung vollflächigen Kleberauftrag aufweist. Es wird aber insbesondere auch vorgeschlagen, dass der dritte Bereich der Kleberbeschichtung einen in der Längsrichtung und in der Querrichtung vollflächigen Kleberauftrag aufweist. Beides hat sich zur Erzielung einer stabilen Verbindung zwischen Schrittabschnitt und Bauchabschnitt bewährt. Dem eingangs geschilderten Problem der Kleberanordnung innerhalb eines Projektionsbereichs von Randbereichen des Absorptionskörpers lässt sich auch bei dem dritten Bereich der Kleberbeschichtung weiter dadurch begegnen, dass dieser Bereich in der Querrichtung nicht bis zu dem jeweiligen Längsrand erstreckt ist, sondern in einem Abstand hiervon endet.

Es erweist sich indessen als ganz besonders vorteilhaft, wenn der zweite Bereich der Kleberbeschichtung einen nicht vollflächigen Kleberauftrag aufweist. Dieser Kleberauftrag könnte in an sich beliebiger Weise partiell, insbesondere nach einem regelmäßigen oder nicht regelmäßigen Muster aus kleberbeschichteten Teilbereichen und kleberfreien Teilbereichen ausgebildet sein. In Weiterbildung dieses Gedankens wird vorgeschlagen, dass der zweite Bereich der Kleberbeschichtung eine Mehrzahl von, insbesondere in Querrichtung erstreckter und insbesondere parallel zueinander verlaufender, Kleberstreifen und kleberfreien Streifen aufweist. Dabei sind Kleberstreifen durch kleberfreie Streifen voneinander beabstandet bzw. wechseln sich Kleberstreifen und kleberfreie Streifen ab. Wenn bei dem zweiten Bereich ein hüftseitiger Kleberstreifen noch von einem kleberfreien Streifen begrenzt ist und/oder ein schrittseitiger Kleberstreifen noch von einem kleberfreien Streifen begrenzt ist, so wird für die Ermittlung der zweiten Längserstreckung des zweiten Bereichs der kleberfreie Streifen noch zu dem zweiten Bereich hinzugerechnet.

Hierbei erweist es sich als vorteilhaft, wenn eine Streifenbreite der Kleberstreifen, also deren Abmessung quer zu deren Erstreckung, wenigstens 1,0 mm, insbesondere wenigstens 1,5 mm, insbesondere höchstens 3,0 mm, insbesondere höchstens 2,5 mm beträgt und/oder wenn eine Breite der kleberfreien Streifen also deren Abmessung quer zu deren Erstreckung wenigstens 2 mm und insbesondere höchstens 10 mm, insbesondere höchstens 8 mm, insbesondere höchstens 6 mm, insbesondere höchstens 5 mm, insbesondere höchstens 4 mm beträgt.

Gerade bei der erfindungsgemäßen Ausbildung des Inkontinenzartikels mit kleberfreien Eckbereichen des Absorptionskörpers im Bauchabschnitt, in dem Sinne, dass in der Projektion dieser Eckbereiche auf den Bauchabschnitt kein Klebermaterial zwischen Schrittabschnitt und Bauchabschnitt vorgesehen ist, erweist es sich als vorteilhaft, wenn in diesem Bereich in der Quer- oder Hüftumfangsrichtung erstreckte Elastifizierungsmittel verlaufen oder ein in sonstiger Weise elastisches oder elastifiziertes Chassismaterial vorhanden ist, welche bzw. welches auch dort elastifizierend wirksam sind. Dieser Vorschlag steht in Abkehr einer an sich bekannten Praxis, Elastifizierungsmittel im Überlappungsbereich mit dem Absorptionskörpers zu schneiden oder in sonstiger Weise zu deaktivieren, um einer Rüschung oder Fältelung des Absorptionskörpers entgegenzuwirken oder jedenfalls nicht zu fördern. Es zeigt sich aber, dass dies zumindest bei der hier beanspruchten erfindungsgemäßen Ausbildung eher nicht vorteilhaft ist, sondern infolge der fehlenden Kleberverbindung in den Eckbereichen zu einem Aufplustern der Eckbereiche des Absorptionskörpers führen könnte. Eine Elastifizierung des Chassismaterials zumindest in Querrichtung außerhalb des zweiten Bereichs der Klebeverbindung wird daher als vorteilhaft angesehen.

Das eingangs erwähnte Problem stellt sich im Überlappungsbereich von Schrittabschnitt und Rückenabschnitt nicht oder in weitaus weniger kritischer Weise. Aufgrund der naturgegebenen Körperform im Gesäßbereich kommt es dort weniger zu verhärteten Materialkonzentrationen. Daher wird für die Ausbildung der Kleberverbindung im Rücken- oder Gesäßbereich insbesondere vorgeschlagen, dass die Kleberverbindung zwischen Schrittabschnitt und Rückenabschnitt einen hüftseitigen vierten Bereich mit einer vierten Längserstreckung L4 in der Längsrichtung und einer vierten Quererstreckung Q4 in der Quer- oder Hüftumfangsrichtung aufweist,
dass die Kleberverbindung zwischen Schrittabschnitt und Rückenabschnitt einen fünften Bereich mit einer fünften Längserstreckung L5 in der Längsrichtung und einer fünften Quererstreckung Q5 in der Quer- oder Hüftumfangsrichtung aufweist, der bezüglich des hüftseitigen vierten Bereichs schrittseitig angeordnet ist,
dass die Kleberverbindung zwischen Schrittabschnitt und Rückenabschnitt einen sechsten Bereich mit einer sechsten Längserstreckung L6 in der Längsrichtung und einer sechsten Quererstreckung Q6 in der Quer- oder Hüftumfangsrichtung aufweist, der bezüglich des fünften Bereichs schrittseitig angeordnet ist, so dass der fünfte Bereich in der Längsrichtung zwischen dem vierten Bereich und dem sechsten Bereich angeordnet ist, und dass der vierte, der fünfte und der sechste Bereich der Kleberverbindung zwischen Schrittabschnitt und Rückenabschnitt im wesentlichen dieselbe Quererstreckung in der Quer- oder Hüftumfangsrichtung aufweisen.

Wiederum erweist es sich als vorteilhaft, wenn der vierte Bereich und/oder der sechste Bereich der Kleberverbindung zwischen Schrittabschnitt und Rückenabschnitt einen in der Längsrichtung und in der Querrichtung vollflächigen Kleberauftrag aufweisen.

Weiter wird insbesondere vorgeschlagen, dass der fünfte Bereich der Kleberbeschichtung einen nicht vollflächigen Kleberauftrag aufweist.

Für diesen nicht flächigen Kleberauftrag wird auch im Rückenbereich insbesondere vorgeschlagen, dass der fünfte Bereich der Kleberverbindung zwischen Schrittabschnitt und Rückenabschnitt eine Mehrzahl von, insbesondere in Querrichtung erstreckter und insbesondere parallel zueinander verlaufender, Kleberstreifen und kleberfreien Streifen aufweist.

Hingegen wird insbesondere vorgeschlagen, dass im Rückenabschnitt in der Quer- oder Hüftumfangsrichtung erstreckte Elastifizierungsmittel verlaufen, die sich auch im Überlappungsbereich von Schrittabschnitt und Bauchabschnitt erstrecken und dort ihrer elastifizierenden Wirkung wenigstens abschnittsweise benommen sind.

Dabei können vorzugsweise in einem schrittseitigen und den Beinöffnungen zugewandten Bereich des Bauchabschnitts und/oder des Rückenabschnitts Elastifizierungsmittel vorgesehen sein, die ausgehend von den beiden Seitennahtbereichen in Richtung auf den Schrittabschnitt, insbesondere in Bezug auf die Längsmittelachse des Inkontinenzartikels, mit zunehmendem Abstand voneinander verlaufen.

An dieser Stelle sei ausgeführt, dass in der vorliegenden Erfindung jegliche Bemessungsangaben hinsichtlich Quererstreckung, Längserstreckung oder Abstand oder Distanz von Abschnitten in eben ausgebreitetem Zustand der den Inkontinenzartikel bildenden Flachmaterialien bestimmt werden, also gegebenenfalls nach Auftrennung der herstellerseitig schon angebrachten Seitennähte, so dass der betreffende Inkontinenzartikel in die in den Figuren beispielhaft dargestellte ebene Konfiguration gebracht werden kann, sofern kein anderer Hinweis auf einen davon abweichenden Zustand angeführt ist. Wenn der Inkontinenzartikel beispielsweise durch fadenförmige Elastifizierungsmittel im sogenannten "Stretchbond-Verfahren" elastifiziert wurde, so werden die Flächenmaterialien, wie in den Figuren angedeutet, derart ausgedehnt betrachtet, wie sie herstellerseitig als Flachmaterialien zugeführt werden oder nachträglich bis zu ihrer natürlichen Ausgangserstreckung ohne Elastifizierungsmittel ausgebreitet und auf eine ebene Fläche aufgelegt werden können. In dieser ebenen Fläche werden dann die Quererstreckungen, Längserstreckungen, Abmessungen, Abstände oder Distanzen ermittelt. Dieser Zustand ergibt sich bei undehnbaren Chassismaterialien auf Vliesbasis oder Folienbasis oder Vlies/Folien-Verbundbasis auf natürliche Weise.

Für die Kleberverbindung wird vorzugsweise eine Kleberbeschichtung mit einem Flächengewicht von 2 - 20 g/m², insbesondere 2 - 15 g/m², weiter insbesondere 2 - 12 g/m², weiter insbesondere 5 - 12 g/m² eingesetzt.

Weiter vorzugsweise weist die Kleberverbindung im ersten und zweiten Bereich eine Kleberbeschichtung mit gleichem Flächengewicht auf.

Weiter vorzugsweise ist im dritten Bereich der Kleberverbindung bei allen Kleberstreifen eine Kleberbeschichtung mit gleichem Flächengewicht vorgesehen.

Weiter vorzugsweise ist im ersten, zweiten und dritten Bereich der Kleberverbindung ein gleiches Flächengewicht vorgesehen.

Weiter vorzugsweise weist die Kleberverbindung im vierten und sechsten Bereich eine Kleberbeschichtung mit gleichem Flächengewicht auf.

Weiter vorzugsweise ist im fünften Bereich der Kleberverbindung bei allen Kleberstreifen eine Kleberbeschichtung mit gleichem Flächengewicht vorgesehen.

Weiter vorzugsweise ist im vierten, fünften und sechsten Bereich der Kleberverbindung ein gleiches Flächengewicht vorgesehen.

Der Inkontinenzartikel in Höschenform ist vorzugsweise ein Erwachsenen-Inkontinenzartikel.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Inkontinenzartikels. In der Zeichnung zeigt:
Figur 1 eine Draufsicht auf einen erfindungsgemäßen Inkontinenzartikel, wobei ein Bauchabschnitt, ein Rückenabschnitt und ein Schrittabschnitt des Inkontinenzartikels noch nicht zur Bildung einer Höschenform verbunden, sondern in flachgelegtem und eben ausgedehntem Zustand dargestellt sind;
Figur 2a, b schematische Schnittansichten nur des Schrittabschnitts im Bereich einer Quermittellinie bzw. in einem Überlappungsbereich von Schrittabschnitt und Bauchabschnitt;
Figur 3 eine vergrößerte Darstellung eines einen bauchseitigen Überlappungsbereich von Schrittabschnitt und Bauchabschnitt umfassenden Teils der Figur 1;
Figur 4 eine vergrößerte Darstellung eines einen rückenseitigen Überlappungsbereich von Schrittabschnitt und Rückenabschnitt umfassenden Teils der Figur 1;

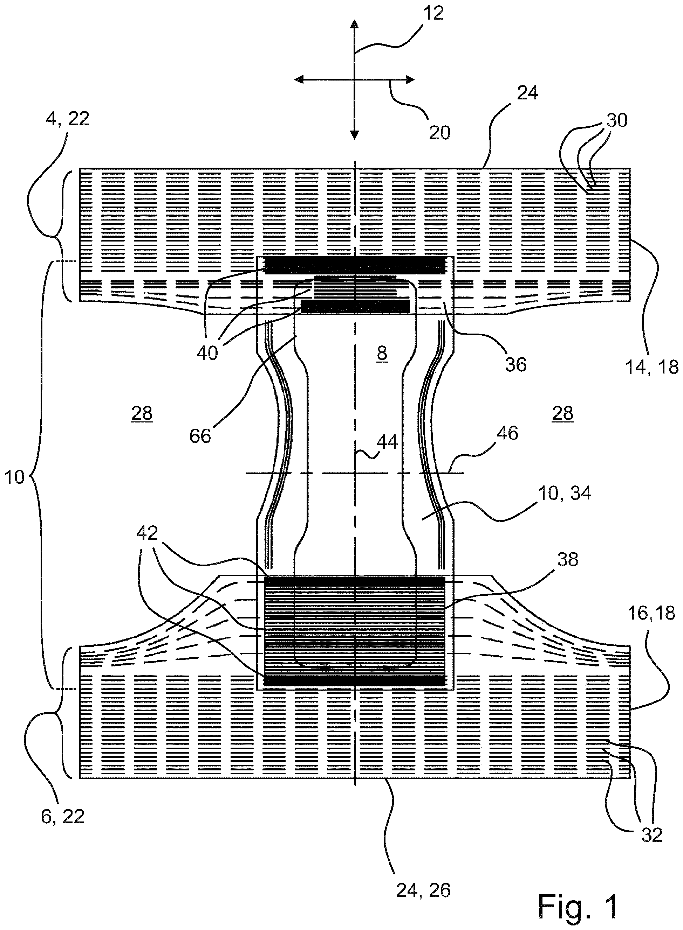

Die Figuren zeigen einen insgesamt mit dem Bezugszeichen 2 bezeichneten Inkontinenzartikel in Höschenform für die Aufnahme fester und flüssiger Körperausscheidungen. Der Inkontinenzartikel 2 ist aus drei weitestgehend unabhängig voneinander fertigbaren Komponenten, nämlich einem vorderen Bauchabschnitt 4, einem hinteren Rückenabschnitt 6 und einem zwischen diesen angeordneten und einen Absorptionskörper 8 aufweisenden Schrittabschnitt 10 gebildet, wobei der Schrittabschnitt 10 in einer Längsrichtung 12 des Inkontinenzartikels 2 erstreckt ist und mit einem wesentlichen Flächenanteil den Bauchabschnitt 4 einerseits und den Rückenabschnitts 6 andererseits überlappt und dort auf noch näher zu beschreibende Weise herstellerseitig mit Bauchabschnitt 4 und Rückenabschnitt 6 unlösbar verbunden ist. Wie aus Figur 1 ersichtlich, führt dies zu einer H-förmigen Grundstruktur des Inkontinenzartikels, wenn Bauchabschnitt 4, Rückenabschnitt 6 und Schrittabschnitt 10 in einem eben ausgebreiteten Zustand betrachtet werden. Die in Figur 1 dargestellten miteinander gefügten Bestandteile werden dann zur Bildung der Höschenform an jeweiligen seitlichen Längsrandabschnitten 14, 16 des Bauchabschnitts 4 und des Rückenabschnitts 6 ebenfalls herstellerseitig miteinander durch übliche Fügeverfahren verbunden, wodurch beidseits Seitennahtbereiche 18 ausgebildet werden. In diesem herstellerseitig gefertigten höschenförmigen Zustand des Inkontinenzartikels 2 bilden der Bauchabschnitt 4 und der Rückenabschnitt 6 ein in Quer- oder Hüftumfangsrichtung 20 durchgehendes Bauch- und Rückenband 22 und definieren so mit ihrem Hüftrand 24 eine in Hüftumfangsrichtung geschlossene Hüftöffnung 26; ferner begrenzen sie zusammen mit dem Schrittabschnitt 10 Beinöffnungen 28, durch welche hindurch der Benutzer den Inkontinenzartikel wie ein Höschen anlegt.

Der Bauchabschnitt 4 und der Rückenabschnitt 6 sind in der Quer- oder Hüftumfangsrichtung 20 elastisch oder elastifiziert ausgebildet. Im hier beispielhaft dargestellten Fall sind im Bauchabschnitt 4 und im Rückenabschnitt 6 eine Vielzahl von in der Quer- oder Hüftumfangsrichtung 20 erstreckten Elastifizierungsmitteln 30, 32 vorgesehen. Es kann sich hierbei um fadenförmige Elastifizierungsmittel, wie Lycra-Fäden, handeln, die in vorgedehntem Zustand, im sogenannten "Stretch-Bond-Verfahren", mit Flachmaterialien des Bauchabschnitts 4 und des Rückenabschnitts 6 verbunden sind. Sie erstrecken sich in der Quer- oder Hüftumfangsrichtung 20 von einem Seitennahtbereich 18 zum anderen. Sie können in an sich bekannter Weise, z.B. durch eine durchgehende Fadenbeleimung oder auch intermittierend mit den jeweiligen Flachmaterialien von Bauchabschnitt 4 und Rückenabschnitt 6 verbunden sein. Durch die in den Figuren angedeutete Erstreckung der Vielzahl von Elastifizierungsmitteln 30, 32 wird eine flächenhafte Elastifizierung von Bauchabschnitt 4 und Rückenabschnitt 6 in der Quer- oder Hüftumfangsrichtung 20 erreicht. In einem schrittseitigen Bereich von Bauchabschnitt 4 und Rückenabschnitt 6 können die Elastifizierungsmittel 30, 32 auch in Richtung auf den Schrittabschnitt 10 sich auffächern, also mit zunehmendem Abstand voneinander erstreckt sein. Anstelle von einzelnen faden- oder bandförmigen Elastifizierungsmitteln oder diese ergänzend könnten auch flächenelastische Flachmaterialien zur Bildung des Bauchabschnitts 4 und/oder des Rückenabschnitts 6 verwendet werden.

Der Schrittabschnitt 10 des Inkontinenzartikels 2 bildet eine den Absorptionskörper 8 aufweisende integrierte Komponente 34, die als solche mit sämtlichen Komponenten gefertigt und dann zugeführt und mit dem Bauchabschnitt 4 und dem Rückenabschnitt 6 verbunden wird. Sie ist in einem bauchseitigen Überlappungsbereich 36 und in einem rückenseitigen Überlappungsbereich 38 mittels einer jeweiligen noch zu erläuternden Kleberverbindung 40, 42 mit dem Bauchabschnitt 4 bzw. mit dem Rückenabschnitt 6 herstellerseitig unlösbar verbunden.

Des weiteren ist eine Längsmittelachse 44 und eine Quermittelachse 46 eingezeichnet, welche den Inkontinenzartikel jeweils halbieren.

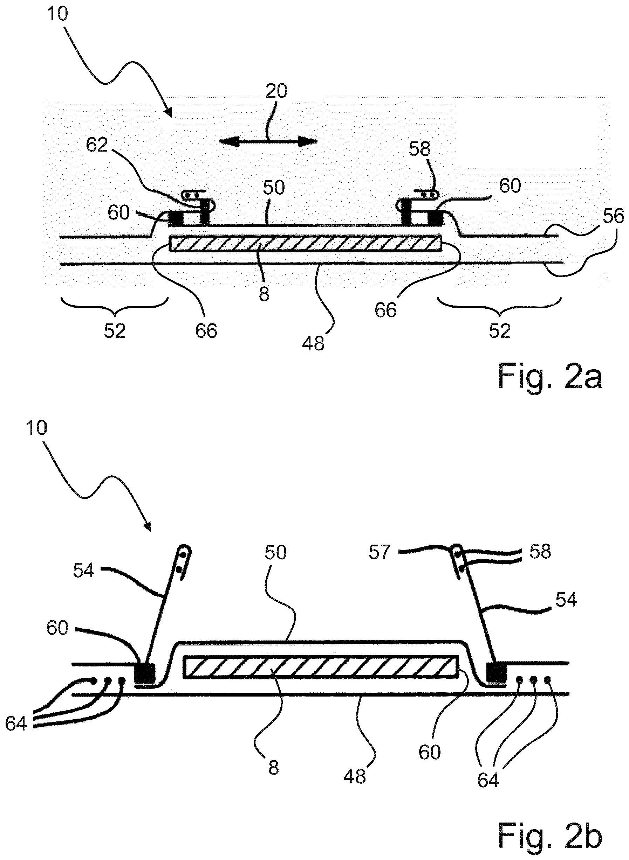

Wie aus Figuren 2a, b ersichtlich umfasst der Schrittabschnitt 10 ein flüssigkeitsundurchlässiges Backsheet-Material 48, das insbesondere von einem atmungsaktiven, jedoch flüssigkeitsdichten Folienmaterial gebildet sein kann, und ein, vorzugsweise auf Vliesbasis beruhendes Topsheet-Material 50. Zwischen dem Backsheet-Material 48 und dem Topsheet-Material 50 ist der Absorptionskörper 8 (nur schematisch dargestellt) angeordnet. Im beispielhaft dargestellten Fall bildet das Backsheet-Material 48 in der Querrichtung 20 einen seitlichen Überhang 52 über den Absorptionskörper 8. Das Topsheet 50 überragt den Absorptionskörper 8 in Querrichtung 20 nur verhältnismäßig geringfügig. Beidseits des Absorptionskörpers 8 ist in Längsrichtung 12 verlaufend jeweils ein aufstehendes Barrieremittel 54 vorgesehen, welches typischerweise als aufstehendes Cuffelement oder Bündchenelement bezeichnet wird und vorzugsweise aus einem hydrophoben, insbesondere flüssigkeitsundurchlässigen Vliesstoffmaterial gebildet ist, welches sich in Querrichtung 16 vorzugsweise bis zu seitlichen Längsrändern 56 des Schrittabschnitts 10 erstreckt. Distale Enden 57 der Barrieremittel 54 sind mit weiteren Elastifizierungsmitteln 58 versehen, welche die Barrieremittel 54 im Gebrauch des Inkontinenzartikels gegen die Hautoberfläche des Benutzers anheben. Die seitlichen Barrieremittel 54 sind über schematisch angedeutete Fixierungen 60, 62 auf das Topsheet-Material 50 beziehungsweise im Bereich ihrer Längsenden auf sich selbst in einer C-förmig gefalteten Konfiguration festgelegt. In der Querrichtung 20 außerhalb des Absorptionskörpers 8 also im Bereich des Überhangs 52 sind ferner Beinelastifizierungsmittel 64 vorgesehen, die vorzugsweise mit einem gewissen Abstand von beidseitigen Längsrändern 66 des materialreichen und damit eher biegesteifen Absorptionskörper 8 verlaufen, um einerseits keine zusätzlichen Dehnungs- oder Verwindungskräfte auf den Absorptionskörper 8 auszuüben, was dessen Absorptionsverhalten nachteilig beeinflussen könnte, und um andererseits einen vom Absorptionskörper 8 weitgehend unbeeinflussten flüssigkeitsdichten Beinabschluss zu realisieren. Diese Beinelastifizierungsmittel 64 enden in Längsrichtung 12 vor dem jeweiligen Überlappungsbereich 36, 38 mit einem Abstand von insbesondere wenigstens 10 mm, vorzugsweise wenigstens 20 mm. Auf diese Weise beeinflussen sie nicht die Querelastifizierung des Bauchabschnitts 4 und des Rückenabschnitts 6 durch die dort vorgesehenen Elastifizierungsmittel 30, 32.

Nachfolgend wird die Fixierung des Schrittabschnitts 10 im bauchseitigen Überlappungsbereich 36 mit dem Bauchabschnitt 4 und im rückenseitigen Überlappungsbereich 38 mit dem Rückenabschnitt 6 beschrieben.

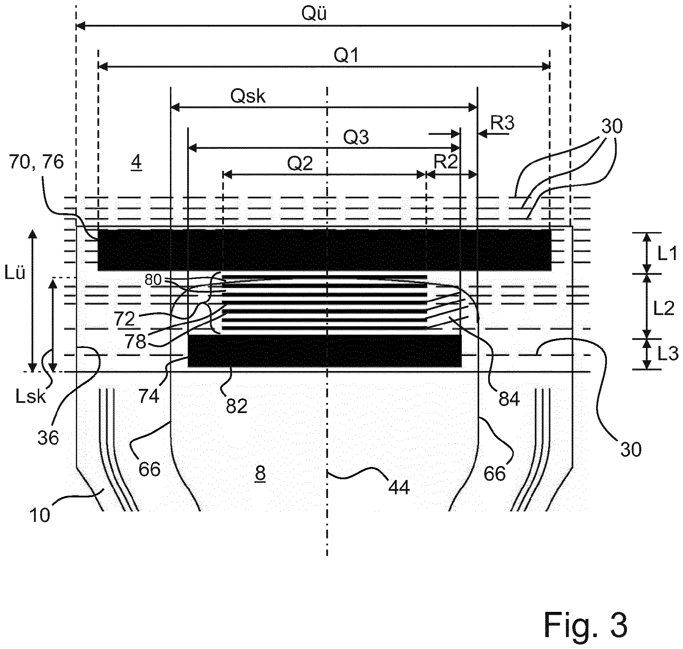

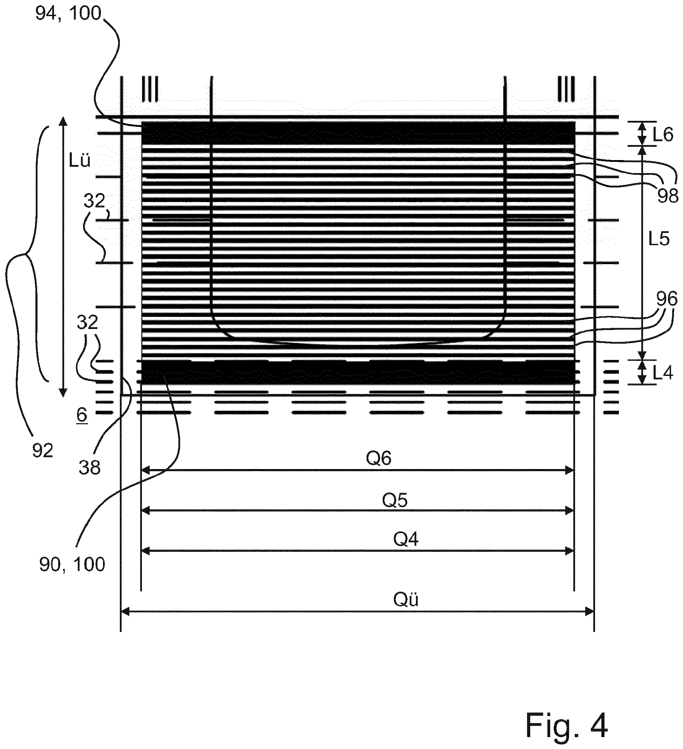

Die Figuren 3 und 4 zeigen einen vergrößerten Ausschnitt des bauchseitigen Überlappungsbereichs 36 bzw. des rückenseitigen Überlappungsbereichs 38. Der Überlappungsbereich 36 von Schrittabschnitt 10 und Bauchabschnitt 4 weist eine Längserstreckung Lü in der Längsrichtung 12 und eine Quererstreckung Qü in der Querrichtung 20 auf.

Die Kleberverbindung 40 zwischen Schrittabschnitt 10 und Bauchabschnitt 4 umfasst einen hüftseitigen ersten Bereich 70 mit einer ersten Längserstreckung L1 in der Längsrichtung 12 und einer ersten Quererstreckung Q1 in der Quer- oder Hüftumfangsrichtung 20 auf.

Des Weiteren weist die Kleberverbindung 40 einen zweiten Bereich 72 mit einer zweiten Längserstreckung L2 in der Längsrichtung 12 und einer zweiten Quererstreckung Q2 in der Quer- oder Hüftumfangsrichtung 20 auf. Ein Querabstand R2 zwischen dem zweiten Bereich 72 und jeweiligen Längsrändern 66 des Absorptionskörpers 8 bei diesem zweiten Bereich 72 ist in Figur 3 ebenfalls dargestellt.

Des Weiteren weist die Kleberverbindung 40 einen dritten Bereich 74 mit einer dritten Längserstreckung L3 in der Längsrichtung 12 und einer dritten Quererstreckung Q3 in der Quer- oder Hüftumfangsrichtung 20 auf. Ein Querabstand R3 zwischen dem dritten Bereich 74 und jeweiligen Längsrändern 66 des Absorptionskörpers 8 bei diesem dritten Bereich 72 ist in Figur 3 ebenfalls dargestellt.

Der zweite Bereich 72 ist dabei in der Längsrichtung 12 zwischen dem hüftseitigen ersten Bereich 70 und dem schrittseitigen dritten Bereich 74 angeordnet.

Der hüftseitige erste Bereich 70 der Kleberverbindung 40 ist beispielhaft von einem flächenhaft durchgehenden vollflächigen Kleberauftrag 76 gebildet, der die Form eines Rechtecks aufweist. Der zweite Bereich 72 der Kleberverbindung ist beispielhaft nicht vollflächig ausgebildet, sondern er ist von einer Mehrzahl von beispielhaft in der Querrichtung 20 erstreckter und parallel zueinander verlaufender Kleberstreifen 78 gebildet. Die Kleberstreifen 78 sind durch kleberfreie Streifen 80 voneinander beabstandet, d.h. Kleberstreifen 78 und kleberfreie Streifen 80 wechseln sich in der Längsrichtung 12 ab. Obschon diese dargestellte Ausführungsform bevorzugt ist, wäre es auch denkbar, dass der zweite Bereich 72 von Kleberstreifen und kleberfreien Streifen gebildet ist, die in einem Winkel zur Querrichtung 20 verlaufen, oder auch von sich überschneidenden Kleberstreifen und kleberfreien Streifen. Im beispielhaft dargestellten Fall liegen in der Längsrichtung 12 außen jeweils ein kleberfreier Streifen 80, welche bei der Bestimmung der zweiten Längserstreckung L2 des zweiten Bereichs 72 hinzugerechnet werden.

Der schrittseitige dritte Bereich 74 ist wiederum von einem vollflächigen Kleberauftrag 82 gebildet, welcher beispielhaft rechteckförmig ausgebildet ist.

Der hüftseitige erste Bereich 70 der Kleberverbindung 40 ist mit seiner gesamten Fläche außerhalb einer Fläche des Absorptionskörpers 8 angeordnet. Der zweite Bereich 72 der Kleberverbindung ist in orthogonaler Projektion auf den Überlappungsbereich 36 betrachtet zumindest in der Querrichtung 20 vollständig innerhalb der Erstreckung des Absorptionskörpers 8 angeordnet. Damit liegen bauchseitige Eckbereiche 84 des Absorptionskörpers 10 wiederum in orthogonaler Projektion auf den Überlappungsbereich 36 betrachtet außerhalb des zweiten Bereichs 72. In Figur 3 ist ein Eckbereich 84 des Absorptionskörpers 8 schraffiert angedeutet.

Der vollflächig ausgebildete dritte Bereich 74 der Kleberverbindung 40 ist in orthogonaler Projektion auf den Überlappungsbereich 36 betrachtet vollständig innerhalb der Erstreckung des Absorptionskörpers 8 angeordnet.

Wie eingangs bereits ausgeführt, ist die erste Quererstreckung
(Q1) des hüftseitigen ersten Bereichs 70 größer ist als die zweite Quererstreckung (Q2) des zweiten Bereichs 72 und größer ist als die dritte Quererstreckung (Q3) des dritten Bereichs 74.

Weiter ist die dritte Quererstreckung (Q3) des dritten Bereichs 74 größer ist als die zweite Quererstreckung (Q2) des zweiten Bereichs 72.

Weiter ist die zweite Quererstreckung (Q2) des zweiten Bereichs 72 geringer ist als eine maximale Quererstreckung Qsk des Absorptionskörpers 8 bei dem zweiten Bereich 72.

Weiter ist der dritte Bereich 74 in orthogonaler Projektion auf den Überlappungsbereich 36 betrachtet in der Längsrichtung 12 und in der Querrichtung 20 vollständig innerhalb einer Erstreckung des Absorptionskörpers 8 angeordnet.

Der zweite Bereich 72 ist in orthogonaler Projektion auf den Überlappungsbereich 36 betrachtet zumindest in der Querrichtung 20 vollständig innerhalb der Erstreckung des Absorptionskörpers 8 angeordnet ist, so dass bauchseitige Eckbereiche 84 des Absorptionskörpers 8 in orthogonaler Projektion auf den Überlappungsbereich 36 außerhalb des zweiten Bereichs 72 liegen.

Die Längserstreckungen und die Quererstreckungen des ersten und zweiten und dritten Bereichs 70, 72, 74 der Kleberverbindung genügen bei einer bevorzugten Ausführungsform des erfindungsgemäßen Inkontinenzartikels den eingangs gegebenen Abmessungen und Relationen zueinander sowie zu einer Quererstreckung Qsk des Absorptionskörpers bei dem jeweiligen ersten 70, zweiten 72 bzw. dritten Bereich 74 bzw. zu einer Längserstreckung Lsk des Absorptionskörpers 8. Wenn der Absorptionskörper 8 wie beispielhaft dargestellt mit einem verrundeten Verlauf der Eckbereiche 84 ausgebildet ist, so wird für die Bestimmung der Quererstreckung Qsk die maximale Quererstreckung des Absorptionskörpers 8 bei dem betreffenden zweiten Bereich 72 herangezogen, so wie dies in Figur 3 eingezeichnet ist. Entsprechendes gilt auch in den anderen Bereichen. Auch zur Bestimmung der ersten, zweiten und dritten Quererstreckung Q1, Q2, Q3 wird jeweils eine maximale Quererstreckung dieser Bereiche 70, 72, 74 herangezogen, sofern keine exakte Rechteckform der Kleberverbindung wie bei dem Ausführungsbeispiel verwendet wird. Eine jeweilige Längserstreckung L1, L2, L3 der drei Bereiche 70, 72, 74 wird jeweils entlang der Längsmittelachse 44, also in einem zentralen Mittelbereich des Inkontinenzartikels, bestimmt, wo die Krafteinleitung auf die Kleberverbindung 40 bzw. 42 naturgemäß am größten ist.

Bei dem erfindungsgemäßen und beispielhaft dargestellten Inkontinenzartikel 2 befinden sich seitliche Eckbereiche 84 des Absorptionskörpers 8 wie ausgeführt in Querrichtung außerhalb des zweiten Bereichs 72 der Kleberbeschichtung.

In orthogonaler Projektion auf den Überlappungsbereich 36 betrachtet befindet sich im Bereich dieser Eckbereiche 84 keine Kleberverbindung zwischen Schrittabschnitt 10 und Bauchabschnitt 4, welche zu einer unangenehmen Konzentration von verhärtetem Material, insbesondere unterstützt durch Bewegungen des Benutzers, im Bereich der Leistenbeuge führt. Um dennoch zu verhindern, dass sich der Absorptionskörper 8 im Bereich dieser Eckbereiche 84 aufplustert oder abhebt, wird vorgeschlagen, dass die dort verlaufenden Elastifizierungsmittel 30 auch im Bereich des Absorptionskörpers 8 oder zumindest im Bereich von dessen Eckbereichen 84 elastisch wirksam bleiben, also dort nicht geschnitten oder in sonstiger Weise ihrer elastifizierenden Wirkung benommen sind.

Figur 4 verdeutlicht die Kleberverbindung 42 im Überlappungsbereich 38 zwischen Schrittabschnitt 10 und Rückenabschnitt 6. Die Kleberverbindung 42 umfasst dort einen hüftseitigen vierten Bereich 90 mit einer vierten Längserstreckung (L4) in der Längsrichtung 12 und einer vierten Quererstreckung (Q4) in der Quer- oder Hüftumfangsrichtung 20. Die Kleberverbindung 42 umfasst weiter einen fünften Bereich 92 mit einer fünften Längserstreckung (L5) in der Längsrichtung 12 und einer fünften Quererstreckung (Q5) in der Quer- oder Hüftumfangsrichtung 20, der bezüglich des hüftseitigen vierten Bereichs (90) schrittseitig angeordnet ist. Die Kleberverbindung 42 umfasst weiter einen sechsten Bereich 94 mit einer sechsten Längserstreckung (L6) in der Längsrichtung 12 und einer sechsten Quererstreckung (Q6) in der Quer- oder Hüftumfangsrichtung 20, der bezüglich des fünften Bereichs 92 schrittseitig angeordnet ist, so dass der fünfte Bereich 92 in der Längsrichtung 12 zwischen dem vierten Bereich 90 und dem sechsten Bereich 94 angeordnet ist.

Wie aus der Figur 4 ersichtlich ist, haben der vierte, der fünfte und der sechste Bereich 90, 92, 94 im Wesentlichen dieselbe Quererstreckung Q4, Q5, Q6.

Der hüftseitige vierte Bereich 90 und der schrittseitige sechste Bereich 94 der Kleberverbindung 42 weisen einen vollflächigen Kleberauftrag 100 auf und sind mit verhältnismäßig geringer Längserstreckung L4 und L6 ausgebildet. Der dazwischen liegende fünfte Bereich 92 erstreckt sich beispielhaft über ungefähr in etwa ¾ der Fläche aller Bereiche. Er umfasst wie der zweite Bereich 72 einen nicht vollflächigen Kleberauftrag in Form einer Mehrzahl von in Querrichtung 20 erstreckter und parallel zueinander verlaufender Kleberstreifen 96 und kleberfreien Streifen 98.

Die in Figur 4 ebenfalls angedeuteten in der Quer- oder Hüftumfangsrichtung 20 verlaufenden Elastifizierungsmittel 32 erstrecken sich wie auch im Bauchabschnitt 4 auch durch den rückenseitigen Überlappungsbereich 38 zwischen Schrittabschnitt 10 und Rückenabschnitt 6. Sie sind aber vorzugsweise zumindest im Bereich des Absorptionskörpers 8 ihrer elastifizierenden Wirkung benommen, indem sie dort geschnitten oder in sonstiger Weise deaktiviert sind, um einem übermäßigen Faltenwurf des Absorptionskörpers 8 im Gesäßbereich entgegenzuwirken.

## Patentansprüche

1. Inkontinenzartikel (2) in Höschenform für die Aufnahme von Körperausscheidungen,
mit einem vorderen Bauchabschnitt (4) und einem hinteren Rückenabschnitt (6), die von separaten und in einer Längsrichtung (12) entlang einer Längsmittelachse (44) voneinander beabstandeten Komponenten gebildet sind, jedoch zur Bildung eines in Quer- oder Hüftumfangsrichtung (20) durchgehenden Bauch- und Rückenbands (22) mit einer in Hüftumfangsrichtung geschlossenen Hüftöffnung (26) an beidseitigen Seitennahtbereichen (18) herstellerseitig miteinander verbunden sind, und
mit einem einen Absorptionskörper (8) aufweisenden Schrittabschnitt (10), der sich in der Längsrichtung (12) zwischen Bauchabschnitt (4) und Rückenabschnitt (6) erstreckt und
mit seiner körperabgewandten Seite an den Bauchabschnitt (4) und an den Rückenabschnitt (6) in einem jeweiligen Überlappungsbereich (36, 38) von Schrittabschnitt (10) und Bauchabschnitt (4) und von Schrittabschnitts (10) und Rückenabschnitt (6) unlösbar angefügt ist,
wobei der Bauchabschnitt (4), der Rückenabschnitt (6) und der Schrittabschnitt (10) gemeinsam Beinöffnungen (28) des Inkontinenzartikels begrenzen,
wobei der Bauchabschnitt (4) und der Rückenabschnitt (6) in der Quer- oder Hüftumfangsrichtung (20) elastisch oder elastifiziert ausgebildet ist,
wobei der Schrittabschnitt (10) im Überlappungsbereich (36) von Schrittabschnitt (10) und Bauchabschnitt (4) und im Überlappungsbereich (38) von Schrittabschnitt (10) und Rückenabschnitt (6) mittels einer Kleberverbindung (40, 42) unlösbar mit dem Bauchabschnitt (4) und mit dem Rückenabschnitt (6) verbunden ist,
wobei der Überlappungsbereich (36) von Schrittabschnitt (10) und Bauchabschnitt(4) eine Längserstreckung (Lü) in der Längsrichtung (12) und eine Quererstreckung (Qü) in der Querrichtung (20) aufweist,
wobei die Kleberverbindung (40) zwischen Schrittabschnitt (10) und Bauchabschnitt (4) einen hüftseitigen ersten Bereich (70) mit einer ersten Längserstreckung (L1) in der Längsrichtung (12) und einer ersten Quererstreckung (Q1) in der Quer- oder Hüftumfangsrichtung (20) aufweist,
wobei die Kleberverbindung (40) zwischen Schrittabschnitt (10) und Bauchabschnitt (4) einen zweiten Bereich (72) mit einer zweiten Längserstreckung (L2) in der Längsrichtung (12) und einer zweiten Quererstreckung (Q2) in der Quer- oder Hüftumfangsrichtung (20) aufweist, der bezüglich des hüftseitigen ersten Bereichs (70) schrittseitig angeordnet ist,
wobei die Kleberverbindung (40) zwischen Schrittabschnitt (10) und Bauchabschnitt (4) einen dritten Bereich (74) mit einer dritten Längserstreckung (L3) in der Längsrichtung (12) und einer dritten Quererstreckung (Q3) in der Quer- oder Hüftumfangsrichtung (20) aufweist, der bezüglich des zweiten Bereichs (72) schrittseitig angeordnet ist, so dass der zweite Bereich (72) in der Längsrichtung (12) zwischen dem ersten Bereich (70) und dem dritten Bereich (74) angeordnet ist,
wobei der erste Bereich (70) in orthogonaler Projektion auf den Überlappungsbereich (36) betrachtet vollständig außerhalb des Absorptionskörpers (8) angeordnet ist,
**dadurch gekennzeichnet,**
**dass** die erste Quererstreckung (Q1) des hüftseitigen ersten Bereichs (70) größer ist als die zweite Quererstreckung (Q2) des zweiten Bereichs (72) und größer ist als die dritte Quererstreckung (Q3) des dritten Bereichs (74),
**dass** die dritte Quererstreckung (Q3) des dritten Bereichs (74) größer ist als die zweite Quererstreckung (Q2) des zweiten Bereichs (72),
**dass** die zweite Quererstreckung (Q2) des zweiten Bereichs (72) geringer ist als eine maximale Quererstreckung (Qsk) des Absorptionskörpers (8) bei dem zweiten Bereich (72), und
**dass** der dritte Bereich (74) in orthogonaler Projektion auf den Überlappungsbereich (36) betrachtet in der Längsrichtung (12) und in der Querrichtung (20) vollständig innerhalb einer Erstreckung des Absorptionskörpers (8) angeordnet ist und
**dass** der zweite Bereich (72) in orthogonaler Projektion auf den Überlappungsbereich (36) betrachtet zumindest in der Querrichtung (20) vollständig innerhalb der Erstreckung des Absorptionskörpers (8) angeordnet ist, so dass bauchseitige Eckbereiche (84) des Absorptionskörpers (8) in orthogonaler Projektion auf den Überlappungsbereich (36) außerhalb des zweiten Bereichs (72) liegen.

2. Inkontinenzartikel nach Anspruch 1, **dadurch gekennzeichnet, dass** in der Querrichtung (20) zwischen dem zweiten Bereich (72) und jeweiligen Längsrändern (66) des Absorptionskörpers (8) beidseits in der Querrichtung (20) ein über die zweite Längserstreckung (L2) des zweiten Bereichs (72) gleichbleibender oder variierender Querabstand (R2) vorgesehen ist und dass wenigstens abschnittsweise über die zweite Längserstreckung (L2) des zweiten Bereichs (72) dieser Querabstand (R2) in der Querrichtung (20) wenigstens 15 mm, insbesondere wenigstens 20 mm, insbesondere wenigstens 25 mm, insbesondere wenigstens 30 mm, und insbesondere höchstens 80 mm, insbesondere höchstens 70 mm, insbesondere höchstens 60 mm, insbesondere höchstens 50 mm beträgt.

3. Inkontinenzartikel nach Anspruch 1 oder 2, **dadurch gekennzeichnet, dass** in der Querrichtung (20) zwischen dem zweiten Bereich (72) und jeweiligen Längsrändern (66) des Absorptionskörpers (8) beidseits in der Querrichtung (20) ein über die zweite Längserstreckung (L2) des zweiten Bereichs (72) gleichbleibender oder variierender Querabstand (R2) vorgesehen ist und dass wenigstens abschnittsweise über die zweite Längserstreckung (L3) des zweiten Bereichs (72) ein Verhältnis dieses Querabstands (R2) zu einer maximalen Quererstreckung (Qsk) des Absorptionskörpers (8) bei dem zweiten Bereich (72) jeweils wenigstens 0,14, insbesondere wenigstens 0,15, insbesondere wenigstens 0,16, und insbesondere höchstens 0,40, insbesondere höchstens 0,30 insbesondere höchstens 0,20 beträgt.

4. Inkontinenzartikel nach Anspruch 1, 2 oder 3, **dadurch gekennzeichnet, dass** in der Querrichtung (20) zwischen dem dritten Bereich (74) und jeweiligen Längsrändern (66) des Absorptionskörpers (8) beidseits in der Querrichtung (20) ein über die dritte Längserstreckung (L3) des dritten Bereichs (74) gleichbleibender oder variierender Querabstand (R3) vorgesehen ist und dass wenigstens abschnittsweise über die dritte Längserstreckung (L3) des dritten Bereichs (74) dieser Querabstand (R3) wenigstens 5 mm, insbesondere wenigstens 8 mm, insbesondere wenigstens 10 mm, insbesondere höchstens 30 mm, insbesondere höchstens 25 mm, insbesondere höchstens 20 mm beträgt.

5. Inkontinenzartikel nach einem oder mehreren der vorstehenden Ansprüche, **dadurch gekennzeichnet, dass** in der Querrichtung (20) zwischen dem dritten Bereich (74) und jeweiligen Längsrändern (66) des Absorptionskörpers (8) beidseits in der Querrichtung (20) ein über die dritte Längserstreckung (L3) des dritten Bereichs (74) gleichbleibender oder variierender Querabstand (R3) vorgesehen ist und dass wenigstens abschnittsweise über die dritte Längserstreckung (L3) des dritten Bereichs (74) ein Verhältnis dieses Querabstands (R3) zu einer maximalen Quererstreckung (Qsk) des Absorptionskörpers (8) bei dem dritten Bereich (74) jeweils wenigstens 0,04, insbesondere wenigstens 0,05, insbesondere wenigstens 0,06, und insbesondere höchstens 0,20, insbesondere höchstens 0,15, insbesondere höchstens 0,10 beträgt.

6. Inkontinenzartikel nach einem oder mehreren der vorstehenden Ansprüche, **dadurch gekennzeichnet, dass** ein Verhältnis der ersten Quererstreckung (Q1) des ersten Bereichs (70) zur zweiten Quererstreckung (Q2) des zweiten Bereichs (72) wenigstens 1,6, insbesondere wenigstens 1,7, insbesondere wenigstens 1,8, insbesondere höchstens 2,5, insbesondere höchstens 2,4, insbesondere höchstens 2,3 beträgt.

7. Inkontinenzartikel nach einem oder mehreren der vorstehenden Ansprüche, **dadurch gekennzeichnet, dass** ein Verhältnis der ersten Quererstreckung (Q1) des ersten Bereichs (70) zur dritten Quererstreckung (Q3) des dritten Bereichs (74) wenigstens 1,2, insbesondere wenigstens 1,3, insbesondere wenigstens 1,4, insbesondere höchstens 2,0, insbesondere höchstens 1,9, insbesondere höchstens 1,8, insbesondere höchstens 1,7 beträgt.

8. Inkontinenzartikel nach einem oder mehreren der vorstehenden Ansprüche, **dadurch gekennzeichnet, dass** ein Verhältnis der zweiten Quererstreckung (Q2) des zweiten Bereichs (72) zur dritten Quererstreckung (Q3) des dritten Bereichs (74) wenigstens 0,6, insbesondere wenigstens 0,7, insbesondere höchstens 0,9, insbesondere höchstens 0,8 beträgt.

9. Inkontinenzartikel nach einem oder mehreren der vorstehenden Ansprüche, **dadurch gekennzeichnet, dass** ein Verhältnis der ersten Längserstreckung (L1) des ersten Bereichs (70), die entlang der Längsmittelachse (44) bestimmt ist, zur Längserstreckung (Lü) des Überlappungsbereichs (36) von Schrittabschnitt (10) und Bauchabschnitt (4), die entlang der Längsmittelachse (44) bestimmt ist, wenigstens 0,10, insbesondere wenigstens 0,12, insbesondere wenigstens 0,15 und insbesondere höchstens 0,50, insbesondere höchstens 0,45, insbesondere höchstens 0,40 beträgt.

10. Inkontinenzartikel nach einem oder mehreren der vorstehenden Ansprüche, **dadurch gekennzeichnet, dass** ein Verhältnis der zweiten Längserstreckung (L2) des zweiten Bereichs (72), die entlang der Längsmittelachse (44) bestimmt ist, zur Längserstreckung (Lü) des Überlappungsbereichs (36) von Schrittabschnitt (10) und Bauchabschnitt (4), die entlang der Längsmittelachse (44) bestimmt ist, wenigstens 0,20, insbesondere wenigstens 0,25, insbesondere wenigstens 0,30 und insbesondere höchstens 0,60, insbesondere höchstens 0,55, insbesondere höchstens 0,50 beträgt.

11. Inkontinenzartikel nach einem oder mehreren der vorstehenden Ansprüche, **dadurch gekennzeichnet, dass** ein Verhältnis der dritten Längserstreckung (L3) des dritten Bereichs (74), die entlang der Längsmittelachse (44) bestimmt ist, zur Längserstreckung (Lü) des Überlappungsbereichs (36) von Schrittabschnitt (10) und Bauchabschnitt (4), die entlang der Längsmittelachse (44) bestimmt ist, wenigstens 0,15, insbesondere wenigstens 0,18, insbesondere wenigstens 0,20 und insbesondere höchstens 0,50, insbesondere höchstens 0,45, insbesondere höchstens 0,40 beträgt.

12. Inkontinenzartikel nach einem oder mehreren der vorstehenden Ansprüche, **dadurch gekennzeichnet, dass** ein Verhältnis der zweiten Längserstreckung (L2) des zweiten Bereichs (72), die entlang der Längsmittelachse (44) bestimmt ist, zur Längserstreckung (Lsk) des Absorptionskörpers (8) im Überlappungsbereich (36) des Schrittabschnitts (10) mit dem Bauchabschnitt (4), die entlang der Längsmittelachse (44) bestimmt ist, wenigstens 0,35, insbesondere wenigstens 0,40, insbesondere wenigstens 0,45 und insbesondere höchstens 0,80, insbesondere höchstens 0,75, insbesondere höchstens 0,70 beträgt.

13. Inkontinenzartikel nach einem oder mehreren der vorstehenden Ansprüche, **dadurch gekennzeichnet, dass** ein Verhältnis der dritten Längserstreckung (L3) des dritten Bereichs (74), die entlang der Längsmittelachse (44) bestimmt ist, zur Längserstreckung (Lsk) des Absorptionskörpers (8) im Überlappungsbereich (36) des Schrittabschnitts (10) mit dem Bauchabschnitt (4), die entlang der Längsmittelachse (44) bestimmt ist, wenigstens 0,25, insbesondere wenigstens 0,30, insbesondere wenigstens 0,35 und insbesondere höchstens 0,65, insbesondere höchstens 0,60, insbesondere höchstens 0,55 beträgt.

14. Inkontinenzartikel nach einem oder mehreren der vorstehenden Ansprüche, **dadurch gekennzeichnet, dass** der erste Bereich (70) und/oder der dritte Bereich (74) der Kleberbeschichtung (40) einen in der Längsrichtung (12) und in der Querrichtung (20) vollflächigen Kleberauftrag (76) aufweisen und/oder dass der zweite Bereich (72) der Kleberbeschichtung (40) einen nicht vollflächigen Kleberauftrag aufweist, wobei insbesondere der zweite Bereich (72) der Kleberbeschichtung (40) eine Mehrzahl von, insbesondere in Querrichtung (20) erstreckter und insbesondere parallel zueinander verlaufender, Kleberstreifen (78) und kleberfreien Streifen (80) aufweist.

15. Inkontinenzartikel nach einem oder mehreren der vorstehenden Ansprüche, **dadurch gekennzeichnet, dass** im Bauchabschnitt (4) in der Quer- oder Hüftumfangsrichtung (20) erstreckte Elastifizierungsmittel (30) verlaufen, die sich auch im Überlappungsbereich (36) von Schrittabschnitt (10) und Bauchabschnitt (4) erstrecken und auch dort elastifizierend wirksam sind.

16. Inkontinenzartikel nach einem oder mehreren der vorstehenden Ansprüche, **dadurch gekennzeichnet, dass** die Kleberverbindung (42) zwischen Schrittabschnitt (10) und Rückenabschnitt (6) einen hüftseitigen vierten Bereich (90) mit einer vierten Längserstreckung (L4) in der Längsrichtung (12) und einer vierten Quererstreckung (Q4) in der Quer- oder Hüftumfangsrichtung (20) aufweist,
dass die Kleberverbindung (42) zwischen Schrittabschnitt (10) und Rückenabschnitt (6) einen fünften Bereich (92) mit einer fünften Längserstreckung (L5) in der Längsrichtung (12) und einer fünften Quererstreckung (Q5) in der Quer- oder Hüftumfangsrichtung (20) aufweist, der bezüglich des hüftseitigen vierten Bereichs (90) schrittseitig angeordnet ist,
dass die Kleberverbindung (42) zwischen Schrittabschnitt (10) und Rückenabschnitt (6) einen sechsten Bereich (94) mit einer sechsten Längserstreckung (L6) in der Längsrichtung (12) und einer sechsten Quererstreckung (Q6) in der Quer- oder Hüftumfangsrichtung (20) aufweist, der bezüglich des fünften Bereichs (92) schrittseitig angeordnet ist, so dass der fünfte Bereich (92) in der Längsrichtung (12) zwischen dem vierten Bereich (90) und dem sechsten Bereich (94) angeordnet ist, und dass der vierte (90), der fünfte (92) und der sechste (94) Bereich der Kleberverbindung (42) zwischen Schrittabschnitt (10) und Rückenabschnitt (6) im wesentlichen dieselbe Quererstreckung (Q4, Q5,Q6) in der Quer- oder Hüftumfangsrichtung (20) aufweisen.

17. Inkontinenzartikel nach Anspruch 16, **dadurch gekennzeichnet, dass** der vierte Bereich (90) und/oder der sechste Bereich (94) der Kleberverbindung (42) zwischen Schrittabschnitt (10) und Rückenabschnitt (6) einen in der Längsrichtung (12) und in der Querrichtung (20) vollflächigen Kleberauftrag (100) aufweisen und/oder dass der fünfte Bereich (92) der Kleberbeschichtung (42) einen nicht vollflächigen Kleberauftrag aufweist, wobei insbesondere der fünfte Bereich (92) der Kleberverbindung (42) zwischen Schrittabschnitt (10) und Rückenabschnitt (6) eine Mehrzahl von, insbesondere in Querrichtung (20) erstreckter und insbesondere parallel zueinander verlaufender, Kleberstreifen (96) und kleberfreien Streifen (98) aufweist.

18. Inkontinenzartikel nach einem der Ansprüche 16-17, **dadurch gekennzeichnet, dass** im Rückenabschnitt (6) in der Quer- oder Hüftumfangsrichtung (20) erstreckte Elastifizierungsmittel (32) verlaufen, die sich auch im Überlappungsbereich (38) von Schrittabschnitt (10) und Rückenabschnitt (6) erstrecken und dort ihrer elastifizierenden Wirkung wenigstens abschnittsweise benommen sind.

## Claims

1. Incontinence article (2) in the form of panties for collecting bodily excretions,
comprising a front stomach portion (4) and a rear back portion (6), which are formed by separate components spaced apart from one another in a longitudinal direction (12) along a longitudinal center axis (44), but are connected to one another by the manufacturer on side seam regions (18) on both sides in order to form a stomach and back strip (22) which is continuous in the transverse direction or hip circumferential direction (20) and has a hip opening (26) closed in the hip circumferential direction, and
comprising a crotch portion (10) having an absorption body (8), which crotch portion extends in the longitudinal direction (12) between the stomach portion (4) and the back portion (6) and
is permanently attached, with the side thereof facing away from the body, to the stomach portion (4) and to the back portion (6) in respective overlap regions (36, 38) of the crotch portion (10) and the stomach portion (4) and of the crotch portion (10) and the back portion (6),
wherein the stomach portion (4), the back portion (6) and the crotch portion (10) together delimit leg openings (28) of the incontinence article,
wherein the stomach portion (4) and the back portion (6) are elastic or elasticized in the transverse direction or hip circumferential direction (20),
wherein the crotch portion (10) is permanently connected, by means of an adhesive connection (40, 42), to the stomach portion (4) and to the back portion (6) in the overlap region (36) of the crotch portion (10) and the stomach portion (4) and in the overlap region (38) of the crotch portion (10) and the back portion (6),
wherein the overlap region (36) of the crotch portion (10) and the stomach portion (4) has a longitudinal extension (Lü) in the longitudinal direction (12) and a transverse extension (Qü) in the transverse direction (20),
wherein the adhesive connection (40) between the crotch portion (10) and the stomach portion (4) has a hip-side first region (70) with a first longitudinal extension (L1) in the longitudinal direction (12) and a first transverse extension (Q1) in the transverse direction or hip circumferential direction (20),
wherein the adhesive connection (40) between the crotch portion (10) and the stomach portion (4) has a second region (72) with a second longitudinal extension (L2) in the longitudinal direction (12) and a second transverse extension (Q2) in the transverse direction or hip circumferential direction (20), which second region is arranged on the crotch side with respect to the hip-side first region (70),
wherein the adhesive connection (40) between the crotch portion (10) and the stomach portion (4) has a third region (74) with a third longitudinal extension (L3) in the longitudinal direction (12) and a third transverse extension (Q3) in the transverse direction or hip circumferential direction (20), which third region is arranged on the crotch side with respect to the second region (72), so that the second region (72) is arranged between the first region (70) and the third region (74) in the longitudinal direction (12),
wherein the first region (70) is arranged completely outside the absorption body (8) when viewed in orthogonal projection onto the overlap region (36),
**characterized**
**in that** the first transverse extension (Q1) of the hip-side first region (70) is greater than the second transverse extension (Q2) of the second region (72) and is greater than the third transverse extension (Q3) of the third region (74),
**in that** the third transverse extension (Q3) of the third region (74) is greater than the second transverse extension (Q2) of the second region (72), in that the second transverse extension (Q2) of the second region (72) is smaller than a maximum transverse extension (Qsk) of the absorption body (8) in the second region (72), and
**in that** the third region (74) is arranged in the longitudinal direction (12) and in the transverse direction (20) completely within an extension of the absorption body (8) when viewed in orthogonal projection onto the overlap region (36), and in that the second region (72) is arranged completely within the extension of the absorption body (8) at least in the transverse direction (20) when viewed in orthogonal projection onto the overlap region (36), so that the stomach-side corner regions (84) of the absorption body (8) are located outside the second region (72) in orthogonal projection onto the overlap region (36).

2. Incontinence article according to claim 1, **characterized in that** a transverse spacing (R2) which remains constant or varies over the second longitudinal extension (L2) of the second region (72) is provided in the transverse direction (20) between the second region (72) and the respective longitudinal edges (66) of the absorption body (8) on both sides in the transverse direction (20), and **in that** over the second longitudinal extension (L2) of the second region (72), at least in portions, this transverse spacing (R2) is at least 15 mm, in particular at least 20 mm, in particular at least 25 mm, in particular at least 30 mm, and in particular at most 80 mm, in particular at most 70 mm, in particular at most 60 mm, in particular at most 50 mm in the transverse direction (20).

3. Incontinence article according to claim 1 or claim 2, **characterized in that** a transverse spacing (R2) which remains constant or varies over the second longitudinal extension (L2) of the second region (72) is provided in the transverse direction (20) between the second region (72) and the respective longitudinal edges (66) of the absorption body (8) on both sides in the transverse direction (20), and **in that** over the second longitudinal extension (L3) of the second region (72), at least in portions, a ratio of this transverse spacing (R2) to a maximum transverse extension (Qsk) of the absorption body (8) in the second region (72) is at least 0.14, in particular at least 0.15, in particular at least 0.16, and in particular at most 0.40, in particular at most 0.30, in particular at most 0.20.

4. Incontinence article according to claim 1, claim 2 or claim 3, **characterized in that** a transverse spacing (R3) which remains constant or varies over the third longitudinal extension (L3) of the third region (74) is provided in the transverse direction (20) between the third region (74) and the respective longitudinal edges (66) of the absorption body (8) on both sides in the transverse direction (20), and **in that** over the third longitudinal extension (L3) of the third region (74), at least in portions, this transverse spacing (R3) is at 5 mm, in particular at least 8 mm, in particular at least 10 mm, in particular at most 30 mm, in particular at most 25 mm, in particular at most 20 mm.

5. Incontinence article according to one of more of the preceding claims, **characterized in that** a transverse spacing (R3) which remains constant or varies over the third longitudinal extension (L3) of the third region (74) is provided in the transverse direction (20) between the third region (74) and the respective longitudinal edges (66) of the absorption body (8) on both sides in the transverse direction (20), and **in that** over the third longitudinal extension (L3) of the second region (74), at least in portions, a ratio of this transverse spacing (R3) to a maximum transverse extension (Qsk) of the absorption body (8) in the third region (74) is at least 0.04, in particular at least 0.05, in particular at least 0.06, and in particular at most 0.20, in particular at most 0.15, in particular at most 0.10.

6. Incontinence article according to one or more of the preceding claims **characterized in that** a ratio of the first transverse extension (Q1) of the first region (70) to the second transverse extension (Q2) of the second region (72) is at least 1.6, in particular at least 1.7, in particular at least 1.8, in particular at most 2.5, in particular at most 2.4, in particular at most 2.3.

7. Incontinence article according to one or more of the preceding claims **characterized in that** a ratio of the first transverse extension (Q1) of the first region (70) to the third transverse extension (Q3) of the third region (74) is at least 1.2, in particular at least 1.3, in particular at least 1.4, in particular at most 2.0, in particular at most 1.9, in particular at most 1.8, in particular at most 1.7.

8. Incontinence article according to one or more of the preceding claims, **characterized in that** a ratio of the second transverse extension (Q2) of the second region (72) to the third transverse extension (Q3) of the third region (74) is at least 0.6, in particular at least 0.7, in particular at most 0.9, in particular at most 0.8.

9. Incontinence article according to one or more of the preceding claims, **characterized in that** a ratio of the first longitudinal extension (L1) of the first region (70), which is determined along the longitudinal center axis (44), to the longitudinal extension (Lü) of the overlap region (36) of the crotch portion (10) and the stomach portion (4), which is determined along the longitudinal center axis (44), is at least 0.10, in particular at least 0.12, in particular at least 0.15 and in particular at most 0.50, in particular at most 0.45, in particular at most 0.40.

10. Incontinence article according to one or more of the preceding claims, **characterized in that** a ratio of the second longitudinal extension (L2) of the second region (72), which is determined along the longitudinal center axis (44), to the longitudinal extension (Lü) of the overlap region (36) of the crotch portion (10) and the stomach portion (4), which is determined along the longitudinal center axis (44), is at least 0.20, in particular at least 0.25, in particular at least 0.30 and in particular at most 0.60, in particular at most 0.55, in particular at most 0.50.

11. Incontinence article according to one or more of the preceding claims, **characterized in that** a ratio of the third longitudinal extension (L3) of the third region (74), which is determined along the longitudinal center axis (44), to the longitudinal extension (Lü) of the overlap region (36) of the crotch portion (10) and the stomach portion (4), which is determined along the longitudinal center axis (44), is at least 0.15, in particular at least 0.18, in particular at least 0.20 and in particular at most 0.50, in particular at most 0.45, in particular at most 0.40.

12. Incontinence article according to one or more of the preceding claims, **characterized in that** a ratio of the second longitudinal extension (L2) of the second region (72), which is determined along the longitudinal center axis (44), to the longitudinal extension (Lsk) of the absorption body (8) in the overlap region (36) of the crotch portion (10) with the stomach portion (4), which is determined along the longitudinal center axis (44), is at least 0.35, in particular at least 0.40, in particular at least 0.45 and in particular at most 0.80, in particular at most 0.75, in particular at most 0.70.

13. Incontinence article according to one or more of the preceding claims **characterized in that** a ratio of the third longitudinal extension (L3) of the third region (74), which is determined along the longitudinal center axis (44), to the longitudinal extension (Lsk) of the absorption body (8) in the overlap region (36) of the crotch portion (10) with the stomach portion (4), which is determined along the longitudinal center axis (44), is at least 0.25, in particular at least 0.30, in particular at least 0.35 and in particular at most 0.65, in particular at most 0.60, in particular at most 0.55.

14. Incontinence article according to one or more of the preceding claims, **characterized in that** the first region (70) and/or the third region (74) of the adhesive coating (40) have a full-surface adhesive application (76) in the longitudinal direction (12) and in the transverse direction (20), and/or **in that** the second region (72) of the adhesive coating (40) has a non-full-surface adhesive application, in particular the second region (72) of the adhesive coating (40) having a plurality of adhesive strips (78) and adhesive-free strips (80) extending in particular in the transverse direction (20) and in particular running in parallel with one another.

15. Incontinence article according to one or more of the preceding claims **characterized in that** elasticizing means (30) extending in the transverse direction or hip circumferential direction (20) run in the stomach portion (4), which elasticizing means also extend in the overlap region (36) of the crotch portion (10) and the stomach portion (4) and also have an elasticizing effect there.

16. Incontinence article according to one or more of the preceding claims **characterized in that** the adhesive connection (42) between the crotch portion (10) and the back portion (6) has a hip-side fourth region (90) with a fourth longitudinal extension (L4) in the longitudinal direction (12) and a fourth transverse extension (Q4) in the transverse direction or hip circumferential direction (20),
**in that** the adhesive connection (42) between the crotch portion (10) and the back portion (6) has a fifth region (92) with a fifth longitudinal extension (L5) in the longitudinal direction (12) and a fifth transverse extension (Q5) in the transverse direction or hip circumferential direction (20), which fifth region which is arranged on the crotch side with respect to the hip-side fourth region (90),
**in that** the adhesive connection (42) between the crotch portion (10) and the stomach portion (6) has a sixth region (94) with a sixth longitudinal extension (L6) in the longitudinal direction (12) and a sixth transverse extension (Q6) in the transverse direction or hip circumferential direction (20), which sixth region is arranged on the crotch side with respect to the fifth region (92), so that the fifth region (92) is arranged between the fourth region (90) and the sixth region (94) in the longitudinal direction (12), and **in that** the fourth (90), the fifth (92) and the sixth (94) region of the adhesive connection (42) between the crotch portion (10) and the back portion (6) have substantially the same transverse extension (Q4, Q5, Q6) in the transverse direction or hip circumferential direction (20).

17. Incontinence article according to claim 16, **characterized in that** the fourth region (90) and/or the sixth region (94) of the adhesive connection (42) between the crotch portion (10) and the back portion (6) have a full-surface adhesive application (100) in the longitudinal direction (12) and in the transverse direction (20), and/or **in that** the fifth region (92) of the adhesive coating (42) has a non-full-surface adhesive application, in particular the fifth region (92) of the adhesive connection (42) between the crotch portion (10) and the back portion (6) having a plurality of adhesive strips (96) and adhesive-free strips (98) extending in particular in the transverse direction (20) and in particular running in parallel with one another.

18. Incontinence article according to any of claims 16-17, **characterized in that** elasticizing means (32) extending in the transverse direction or hip circumferential direction (20) run in the back portion (6), which elasticizing means also extend in the overlap region (38) of the crotch portion (10) and the back portion (6) and are deprived of their elasticizing effect there at least in portions.

## Revendications

1. Article d'incontinence (2) sous forme de culotte, destiné à recueillir les excrétions corporelles,
comprenant une portion ventrale avant (4) et une portion dorsale arrière (6) qui sont constituées par des composants séparés et espacés l'un de l'autre dans une direction longitudinale (9) suivant un axe central longitudinal (44), mais qui, pour la formation d'une bande ventrale et dorsale (22) continue dans le sens transversal ou dans le sens du tour de hanches (20), avec une ouverture de hanche (18) fermée dans le sens du tour de hanches, sont reliés entre eux par le fabricant au niveau de zones de couture latérale (18) situées de part et d'autre, et
comprenant une portion d'entrejambe (10) qui présente un corps absorbant (8) et s'étend dans la direction longitudinale (12) entre la portion ventrale (4) et la portion dorsale (6) et
qui est rapportée de manière inamovible, par son côté montrant dans la direction opposé au corps, sur la portion ventrale (4) et sur la portion dorsale (6) dans une zone de chevauchement (36, 38) respective des portions d'entrejambe (10) et ventrale (4) et des portions d'entrejambe (10) et dorsale (6),
dans lequel ladite portion ventrale (4), ladite portion dorsale (6) et ladite portion d'entrejambe (10) délimitent ensemble des ouvertures de jambe (28) de l'article d'incontinence,
dans lequel la portion ventrale (4) et la portion dorsale (6) sont réalisées de manière élastique ou élastifiée dans le sens transversal ou dans le sens du tour de hanches (20),
dans lequel, dans la zone de chevauchement (36) de la portion d'entrejambe (10) et de la portion ventrale (4) ainsi que dans la zone de chevauchement (38) de la portion d'entrejambe (10) et de la portion dorsale (6), la portion d'entrejambe (10) est reliée de manière inamovible au moyen d'une liaison de colle (40, 42) à la portion ventrale (4) et à la portion dorsale (6),
dans lequel la zone de chevauchement (36) de la portion d'entrejambe (10) et de la portion ventrale (4) présente une extension longitudinale (Lü) dans la direction longitudinale (12) et une extension transversale (Qü) dans la direction transversale (20),
dans lequel la liaison de colle (40) entre la portion d'entrejambe (10) et la portion ventrale (4) présente une première zone côté hanche (70) avec une première extension longitudinale (L1) dans la direction longitudinale (12) et une première extension transversale (Q1) dans le sens transversal ou dans le sens du tour de hanches (20),
dans lequel la liaison de colle (40) entre la portion d'entrejambe (10) et la portion ventrale (4) présente une deuxième zone (72) avec une deuxième extension longitudinale (L2) dans la direction longitudinale (12) et une deuxième extension transversale (Q2) dans le sens transversal ou dans le sens du tour de hanches (20), qui est disposée côté entrejambe par rapport à la première zone côté hanche (70),
dans lequel la liaison de colle (40) entre la portion d'entrejambe (10) et la portion ventrale (4) présente une troisième zone (74) avec une troisième extension longitudinale (L3) dans la direction longitudinale (12) et une troisième extension transversale (Q3) dans le sens transversal ou dans le sens du tour de hanches (20), qui est disposée côté entrejambe par rapport à la deuxième zone (72) de sorte que la deuxième zone (72) est disposée dans la direction longitudinale (12) entre la première zone (70) et la troisième zone (74),
dans lequel la première zone (70) est disposée complètement à l'extérieur du corps absorbant (8), vue en projection orthogonale sur la zone de chevauchement (36),
**caractérisé par le fait**
**que** la première extension transversale (Q1) de la première zone côté hanche (70) est supérieure à la deuxième extension transversale (Q2) de la deuxième zone (72) et est supérieure à la troisième extension transversale (Q3) de la troisième zone (74),
**que** la troisième extension transversale (Q3) de la troisième zone (74) est supérieure à la deuxième extension transversale (Q2) de la deuxième zone (72),
**que** la deuxième extension transversale (Q2) de la deuxième zone (72) est inférieure à une extension transversale maximale (Qsk) du corps absorbant (8) dans la deuxième zone (72), et
**que**, vue en projection orthogonale sur la zone de chevauchement (36), la troisième zone (74) est disposée complètement à l'intérieur d'une extension du corps absorbant (8) dans la direction longitudinale (12) et dans la direction transversale (20), et
**que**, vue en projection orthogonale sur la zone de chevauchement (36), la deuxième zone (72) est disposée complètement à l'intérieur de l'extension du corps absorbant (8), au moins dans la direction transversale (20), de sorte que des zones d'angle côté ventre (84) du corps absorbant (8) sont situées à l'extérieur de la deuxième zone (72) en projection orthogonale sur la zone de chevauchement (36).

2. Article d'incontinence selon la revendication 1, **caractérisé par le fait que** dans la direction transversale (20) entre la deuxième zone (72) et des bords longitudinaux respectifs (66) du corps absorbant (8), de part et d'autre dans la direction transversale (20), est prévue une distance transversale (R2) constante ou variable sur la deuxième extension longitudinale (L2) de la deuxième zone (72), et que, au moins par sections sur la deuxième extension longitudinale (L2) de la deuxième zone (72), cette distance transversale (R2) est d'au moins 15 mm, en particulier d'au moins 20 mm, en particulier d'au moins 25 mm, en particulier d'au moins 30 mm, et en particulier de 80 mm tout au plus, en particulier de 70 mm tout au plus, en particulier de 60 mm tout au plus, en particulier de 50 mm tout au plus, dans la direction transversale (20).

3. Article d'incontinence selon la revendication 1 ou 2, **caractérisé par le fait que** dans la direction transversale (20) entre la deuxième zone (72) et des bords longitudinaux respectifs (66) du corps absorbant (8), de part et d'autre dans la direction transversale (20), est prévue une distance transversale (R2) constante ou variable sur la deuxième extension longitudinale (L2) de la deuxième zone (72), et que, au moins par sections sur la deuxième extension longitudinale (L3) de la deuxième zone (72), un rapport de cette distance transversale (R2) à une extension transversale maximale (Qsk) du corps absorbant (8) dans la deuxième zone (72) est respectivement d'au moins 0,14, en particulier d'au moins 0,15, en particulier d'au moins 0,16, et en particulier de 0,40 tout au plus, en particulier de 0,30 tout au plus, en particulier de 0,20 tout au plus.

4. Article d'incontinence selon la revendication 1, 2 ou 3, **caractérisé par le fait que** dans la direction transversale (20) entre la troisième zone (74) et des bords longitudinaux respectifs (66) du corps absorbant (8), de part et d'autre dans la direction transversale (20), est prévue une distance transversale (R3) constante ou variable sur la troisième extension longitudinale (L3) de la troisième zone (74), et que, au moins par sections sur la troisième extension longitudinale (L3) de la troisième zone (74), cette distance transversale (R3) est d'au moins 5 mm, en particulier d'au moins 8 mm, en particulier d'au moins 10 mm, en particulier de 30 mm tout au plus, en particulier de 25 mm tout au plus, en particulier de 20 mm tout au plus.

5. Article d'incontinence selon l'une ou plusieurs des revendications précédentes, **caractérisé par le fait que** dans la direction transversale (20) entre la troisième zone (74) et des bords longitudinaux respectifs (66) du corps absorbant (8), de part et d'autre dans la direction transversale (20), est prévue une distance transversale (R3) constante ou variable sur la troisième extension longitudinale (L3) de la troisième zone (74), et que, au moins par sections sur la troisième extension longitudinale (L3) de la troisième zone (74), un rapport de cette distance transversale (R3) à une extension transversale maximale (Qsk) du corps absorbant (8) dans la troisième zone (74) est respectivement d'au moins 0,04, en particulier d'au moins 0,05, en particulier d'au moins 0,06, et en particulier de 0,20 tout au plus, en particulier de 0,15 tout au plus, en particulier de 0,10 tout au plus.

6. Article d'incontinence selon l'une ou plusieurs des revendications précédentes, **caractérisé par le fait qu'**un rapport de la première extension transversale (Q1) de la première zone (70) à la deuxième extension transversale (Q2) de la deuxième zone (72) est d'au moins 1,6, en particulier d'au moins 1,7, en particulier d'au moins 1,8, en particulier de 2,5 tout au plus, en particulier de 2,4 tout au plus, en particulier de 2,3 tout au plus.

7. Article d'incontinence selon l'une ou plusieurs des revendications précédentes, **caractérisé par le fait qu'**un rapport de la première extension transversale (Q1) de la première zone (70) à la troisième extension transversale (Q3) de la troisième zone (74) est d'au moins 1,2, en particulier d'au moins 1,3, en particulier d'au moins 1,4, en particulier de 2,0 tout au plus, en particulier de 1,9 tout au plus, en particulier de 1,8 tout au plus, en particulier de 1,7 tout au plus.

8. Article d'incontinence selon l'une ou plusieurs des revendications précédentes, **caractérisé par le fait qu'**un rapport de la deuxième extension transversale (Q2) de la deuxième zone (72) à la troisième extension transversale (Q3) de la troisième zone (74) est d'au moins 0,6, en particulier d'au moins 0,7, en particulier de 0,9 tout au plus, en particulier de 0,8 tout au plus.

9. Article d'incontinence selon l'une ou plusieurs des revendications précédentes, **caractérisé par le fait qu'**un rapport de la première extension longitudinale (L1) de la première zone (70), qui est déterminée suivant l'axe central longitudinal (44), à l'extension longitudinale (Lü) de la zone de chevauchement (36) des portions d'entrejambe (10) et ventrale (4), qui est déterminée suivant l'axe central longitudinal (44), est d'au moins 0,10, en particulier d'au moins 0,12, en particulier d'au moins 0,15 et en particulier de 0,50 tout au plus, en particulier de 0,45 tout au plus, en particulier de 0,40 tout au plus.

10. Article d'incontinence selon l'une ou plusieurs des revendications précédentes, **caractérisé par le fait qu'**un rapport de la deuxième extension longitudinale (L2) de la deuxième zone (72), qui est déterminée suivant l'axe central longitudinal (44), à l'extension longitudinale (Lü) de la zone de chevauchement (36) des portions d'entrejambe (10) et ventrale (4), qui est déterminée suivant l'axe central longitudinal (44), est d'au moins 0,20, en particulier d'au moins 0,25, en particulier d'au moins 0,30 et en particulier de 0,60 tout au plus, en particulier de 0,55 tout au plus, en particulier de 0,50 tout au plus.

11. Article d'incontinence selon l'une ou plusieurs des revendications précédentes, **caractérisé par le fait qu'**un rapport de la troisième extension longitudinale (L3) de la troisième zone (74), qui est déterminée suivant l'axe central longitudinal (44), à l'extension longitudinale (Lü) de la zone de chevauchement (36) des portions d'entrejambe (10) et ventrale (4), qui est déterminée suivant l'axe central longitudinal (44), est d'au moins 0,15, en particulier d'au moins 0,18, en particulier d'au moins 0,20 et en particulier de 0,50 tout au plus, en particulier de 0,45 tout au plus, en particulier de 0,40 tout au plus.

12. Article d'incontinence selon l'une ou plusieurs des revendications précédentes, **caractérisé par le fait qu'**un rapport de la deuxième extension longitudinale (L2) de la deuxième zone (72), qui est déterminée suivant l'axe central longitudinal (44), à l'extension longitudinale (Lsk) du corps absorbant (8) dans la zone de chevauchement (36) des portions d'entrejambe (10) et ventrale (4), qui est déterminée suivant l'axe central longitudinal (44), est d'au moins 0,35, en particulier d'au moins 0,40, en particulier d'au moins 0,45 et en particulier de 0,80 tout au plus, en particulier de 0,75 tout au plus, en particulier de 0,70 tout au plus.

13. Article d'incontinence selon l'une ou plusieurs des revendications précédentes, **caractérisé par le fait qu'**un rapport de la troisième extension longitudinale (L3) de la troisième zone (74), qui est déterminée suivant l'axe central longitudinal (44), à l'extension longitudinale (Lsk) du corps absorbant (8) dans la zone de chevauchement (36) des portions d'entrejambe (10) et ventrale (4), qui est déterminée suivant l'axe central longitudinal (44), est d'au moins 0,25, en particulier d'au moins 0,30, en particulier d'au moins 0,35 et en particulier de 0,65 tout au plus, en particulier de 0,60 tout au plus, en particulier de 0,55 tout au plus.

14. Article d'incontinence selon l'une ou plusieurs des revendications précédentes, **caractérisé par le fait que** la première zone (70) et/ou la troisième zone (74) du revêtement de colle (40) présente une application de colle (76) sur toute la surface dans la direction longitudinale (12) et dans la direction transversale (20), et/ou que la deuxième zone (72) du revêtement de colle (40) présente une application de colle non pas sur toute la surface, dans lequel en particulier la deuxième zone (72) du revêtement de colle (40) présente une pluralité de bandes de colle (78) et de bandes exemptes de colle (80) s'étendant en particulier dans la direction transversale (20) et en particulier parallèlement les unes aux autres.

15. Article d'incontinence selon l'une ou plusieurs des revendications précédentes, **caractérisé par le fait que** des moyens d'élastification (30) s'étendant dans le sens transversal ou dans le sens du tour de hanches (20) s'étendent dans la portion ventrale (4), qui s'étendent également dans la zone de chevauchement (36) des portions d'entrejambe (10) et ventrale (4) et y ont également un effet élastifiant.

16. Article d'incontinence selon l'une ou plusieurs des revendications précédentes, **caractérisé par le fait que** la liaison de colle (42) entre la portion d'entrejambe (10) et la portion dorsale (6) présente une quatrième zone côté hanche (90) avec une quatrième extension longitudinale (L4) dans la direction longitudinale (12) et une quatrième extension transversale (Q4) dans le sens transversal ou dans le sens du tour de hanche (20),
que la liaison de colle (42) entre la portion d'entrejambe (10) et la portion dorsale (6) présente une cinquième zone (92) avec une cinquième extension longitudinale (L5) dans la direction longitudinale (12) et une cinquième extension transversale (Q5) dans le sens transversal ou dans le sens du tour de hanches (20), qui est disposée côté entrejambe par rapport à la quatrième zone côté hanche (90),
que la liaison de colle (42) entre la portion d'entrejambe (10) et la portion dorsale (6) présente une sixième zone (94) avec une sixième extension longitudinale (L6) dans la direction longitudinale (12) et une sixième extension transversale (Q6) dans le sens transversal ou dans le sens du tour de hanches (20), qui est disposée côté entrejambe par rapport à la cinquième zone (92) de sorte que la cinquième zone (92) est disposée dans la direction longitudinale (12) entre la quatrième zone (90) et la sixième zone (94), et que les quatrième (90), cinquième (92) et sixième (94) zones de la liaison de colle (42) entre la portion d'entrejambe (10) et la portion dorsale (6) présentent pour l'essentiel la même extension transversale (Q4, Q5, Q6) dans le sens transversal ou dans le sens du tour de hanches (20).

17. Article d'incontinence selon la revendication 16, **caractérisé par le fait que** la quatrième zone (90) et/ou la sixième zone (94) de la liaison de colle (42) entre la portion d'entrejambe (10) et la portion dorsale (6) présente une application de colle (100) sur toute la surface dans la direction longitudinale (12) et dans la direction transversale (20) et/ou que la cinquième zone (92) du revêtement de colle (42) présente une application de colle non pas sur toute la surface, dans lequel en particulier la cinquième zone (92) de la liaison de colle (42) entre la portion d'entrejambe (10) et la portion dorsale (6) présente une pluralité de bandes de colle (96) et de bandes exemptes de colle (98) s'étendant en particulier dans la direction transversale (20) et en particulier parallèlement les une aux autres.

18. Article d'incontinence selon l'une quelconque des revendications 16 à 17, **caractérisé par le fait que** des moyens d'élastification (32) s'étendant dans le sens transversal ou dans le sens du tour de hanches (20) s'étendent dans la portion dorsale (6), qui s'étendent également dans la zone de chevauchement (38) des portions d'entrejambe (10) et dorsale (6) et y sont dépourvus, au moins par sections, de leur effet élastifiant.
